# EUROPÄISCHE PATENTANMELDUNG

(11) **EP 4 793 956 A1**
(43) Veröffentlichungstag der Anmeldung: **19.08.2026**
(21) Anmeldenummer: 25213359.0
(22) Anmeldetag: 04.11.2025
(51) Int. Cl.: G16C 20/30, G16C 20/70, G16C 60/00

(54) **ENTWICKLUNGSVERFAHREN ZUR REZEPTIERUNG VON KOMPOSITMASSEN UND PRÄDIKTIVER VORHERSAGE VON EIGENSCHAFTEN DARAUS HERGESTELLTER GIESSWERKSTÜCKE**

(30) Priorität: 17.02.2025 DE 102025105870
(71) Anmelder: Gebrüder Dorfner GmbH & Co. KG, 92242 Hirschau (DE)
(72) Erfinder: Colelli, Vanessa, 92224 Amberg (DE); Süß, Marco, 92431 Neunburg vorm Wald (DE)
(74) Vertreter: Hannke Bittner & Partner mbB Regensburg

(57) **Zusammenfassung**

Die vorliegende Erfindung betrifft ein Verfahren zur, insbesondere prädiktiven, Erzeugung eines ersten Zusammensetzungsdatensatzes (ZD1), welcher Daten für die Rezeptierung einer ersten Füllstoffzusammensetzung (FM₁) und/oder einer ersten Kompositmasse (KM₁) für die Herstellung eines ersten Gießwerkstücks (GWS₁) umfasst, mit den Schritten:
- Erfassung von mindestens zwei Zusammensetzungsdatensätzen (ZD₂, ... ZDₙ) zu mindestens einer Füllstoffzusammensetzung (FM₂, ... FMₙ) und/oder einer Kompositmasse (KM₂, ... KMₙ) und/oder einem Gießwerkstück (GWS₂, ... GWSₙ), wobei Zusammensetzungsdatensätze (ZD₂, ... ZDₙ) wenigstens eine Analysegröße (AGₓ) und eine Anteilsgröße (N_{Ax}) einer Komponente (Aₓ) umfasst, und
- Computer-implementierte Ermittlung von wenigstens einem Wert des ersten Zusammensetzungsdatensatzes (ZD₁) auf Grundlage des zweiten Zusammensetzungsdatensatzes (ZD₂) und mindestens eines weiteren Zusammensetzungsdatensatzes (ZD₃, ... ZDₙ).
Außerdem betrifft der Erfindung ein Computer-implementiertes Verfahren zur Erzeugung eines trainierten Kompositmassenrezeptierungsmodells maschinellen Lernens zur Ermittlung wenigstens einer ersten Eigenschaft (E_{1[FM1]} - E_{n[FM1]}; E_{1[KM1]} - E_{n[KM1]}; E_{1[GWS1]} - E_{n[GWS1]}) einer ersten Füllstoffzusammensetzung (FM₁) und/oder der ersten Kompositmasse (KM₁) und/oder eines ersten daraus herstellbaren Gießwerkstück (GWS₁) auf Basis einer Verwendung von Zusammensetzungsdatensätzen (ZD₂ - ZDₙ), sowie eine Kompositmassenrezept-Ermittlungsvorrichtung.

## Beschreibung

Die vorliegende Erfindung betrifft ein Verfahren zur Erzeugung eines ersten Zusammensetzungsdatensatzes, welcher Daten für die Rezeptierung eines Füllstoffes und/oder einer ersten Kompositmasse für die Herstellung eines ersten Gießwerkstück umfasst. Weiterhin betrifft die Erfindung eine Füllstoff- und/oder Kompositmassenrezept-Ermittlungsvorrichtung zur, insbesondere prädiktiven, Ermittlung wenigstens einer Eigenschaft eines ersten Füllstoffes, einer ersten Kompositmasse und/oder einem ersten Gießwerkstück. Weiterhin bezieht sich die Erfindung auf ein Verfahren zur Erzeugung eines (trainierten) Füllstoff- und/oder Kompositmassenrezeptierungsmodells maschinellen Lernens zur Ermittlung wenigstens einer ersten Eigenschaft einer ersten Füllstofflösung und/oder einer ersten Kompositmasse und/oder eines ersten daraus herstellbaren Gießwerkstück auf Basis einer Verwendung von Zusammensetzungsdatensätzen und eine Verwendung dieses Modells zur Bestimmung bzw. zur Ermittlung wenigstens einer für wenigstens eine Eigenschaft einer ersten Füllstofflösung und/oder Kompositmasse und/oder einem ersten Gießwerkstück charakteristischen Analysegröße.

Aus dem Stand der Technik sind eine Vielzahl verschiedener Füllstoffe sowie Kompositmassen und daraus hergestellter Gießwerkstück bekannt. Diese sind für die jeweiligen Aufgaben und Anwendungsszenarien angepasst. Bekannte Anwendungen für Kompositmassen und daraus hergestellter Gießwerkstücke sind beispielsweise Küchenspülen, Sanitärprodukte wie Waschbecken, Duschtassen und -kabinen, Badewannen, Toiletten und Bidets. Außerdem finden sie Verwendung im Bauwesen, im Möbel- und Automobilbau. So werden aus Kompositmassen hergestellte Gießwerkstücke beispielsweise als Arbeitsplatten, Fußboden oder Wandverkleidungen, Profile zur elektrischen Isolation, Möbel(-teile), Schreibgeräte, Urnen, Haushaltsgeräte, Türinnenverkleidungen und Hutablagen verwendet.

Aus dem Stand der Technik ist bereits eine große Anzahl verschiedener Verbundwerkstoffe bekannt. Diese bestehen in der Regel aus partikulären anorganischen Füllstoffen, wie Mineralien (z.B. Quarzsand), die in ein organisches Bindemittel (z.B. Polyester oder Acryl) eingebettet sind und so den Verbundwerkstoff bilden.

Neben diesen Verbundwerkstoffen mit mineralischen Füllstoffen sind außerdem Verbundwerkstoffe bekannt, bei denen synthetische Füllstoffe (z.B. ATH) in organische Bindemittel eingebettet werden (z.B. bekannt als "Solid Surface Material"). Daneben ist auch bekannt, dass Mischungen aus unterschiedlichen Mineralien als Füllstoff in einem Verbundwerkstoff eingesetzt werden können.

Füllstoffhaltige Polymere werden seit vielen Jahrzehnten im industriellen Maßstab hergestellt und verwendet. Für wie oben beschriebene Verbundwerkstoffe werden, beispielsweise als Polymere Thermoplaste, Duroplaste oder Elastomere oder Mischungen dieser drei Gruppen in verschiedenen Verhältnissen verwendet.

Die Polymere bzw. die diese Polymere enthaltenden Kompositmassen können Additive enthalten, um in einer Zusammensetzung bestimmte Eigenschaften zu beeinflussen, z.B. Rheologie, Elastizität, Bruchverhalten, Klimabeständigkeit. Die Additive können organisch oder anorganisch sein oder Mischungen dieser sein.

Weiterhin sind Zusammensetzungen für Verbundwerkstoffe bekannt, bei denen die Füllstofffraktion Additive enthalten, um bestimmte Eigenschaften der Füllstoffe selbst oder deren Wirkung im Polymer beeinflussen zu können, z.B. Rheologie, Elastizität, Bruchverhalten, Klimabeständigkeit, Einarbeitung, Verarbeitung, Dauerhaftigkeit, Farbe sowie Kratz- und Abriebbeständigkeit nach DIN EN 13310 usw.. Zur Farbgebung können Farbstoffe oder auch Pigmente eingesetzt werden. Diese Additive können organisch oder anorganisch sein oder Mischungen organischer und anorganischer Additive. Die Additive können als lose Mischung mit anderen Füllstoffen vorliegen oder physikalisch, mechanisch oder chemisch mit dem Füllstoff verbunden sein. Es ist auch eine physikalische oder chemische Bindung mit dem Polymer möglich.

Aus dem Stand der Technik sind z.B. acrylbasierte füllstoffhaltige (z.B. Quarz, ATH, etc.) flächige Formteile bekannt, welche mittels einem Thermoformverfahren zu einem Formteil gefertigt werden, welche z.B. als Küchenspüle, vor allem aber als Waschbecken, Duschtasse oder Badewanne Verwendung finden. Es sind neben Quarz und ATH auch die Verwendung einiger anderer Füllstoffe und Additive möglich.

Weiterhin ist aus dem Stand der Technik das sogenannte Gelcoat-Verfahren bekannt. Hierzu wird eine Negativform mit einer ersten Harzschicht besprüht, die auch füllstoffhaltig sein kann und welche beim späteren Formteil die Sichtseite bildet. Diese erste Schicht wird danach mit einer zweiten füllstoffhaltigen Harzschicht hinterfüllt. Beide Polymerschichten können die oben genannten Füllstoffe und Additive enthalten.

Aus dem Stand der Technik ist schließlich auch das sogenannte Mineralgussverfahren, auch als Solid-Surface-Verfahren bezeichnet, bekannt. Hier werden füllstoffhaltige Polymere mittels Druckförderung in ein Formteil injiziert und härten dort teils katalytisch, teils thermisch induziert aus. Als Füllstoffe werden vorwiegend CaCO₃ und ATH eingesetzt, sowie Additive und ggf. farbgebende Bestandteile.

Daneben können Kompositmassen (in Abhängigkeit von deren Eigenschaften und Bestandteilen) auch im Spritzgussverfahren oder im 3D-Druck eingesetzt werden. Derartige Anwendungen sind bisher jedoch Nischenprodukten vorbehalten.

Die meisten der bekannten Verfahren haben gemeinsam, dass die partikulären Bestandteile mit a) einem Harz (Sirup) aus PMMA und MMA versetzt und unter Druck und erhöhter Temperatur in Formteilen ausgehärtet werden oder b) mit Polyester in Gießmaschinen zu Formteilen gegossen und mithilfe von Härteradditiven ausgehärtet werden. Das bei b) verwendete Polyesterharz kann optional auch einen gewissen Teil an MMA (Methacrylsäuremethylester, Methylmethacrylat) enthalten. Der in a) genannte Sirup kann auch Isobornylmethacrylat (I-BOMA) und Cyclohexylmethacrylat (CHMA) sowie Ethylenglycoldimethylacrylat (EGDMA), 1,6-Hexandioldimethacrylat (1,6-HDDMA) und Triethylenglycoldimethylacrylat (TRGDMA) enthalten, letztere zwei in der Regel eingesetzt als Quervernetzer.

Sowohl bei den eingesetzten Polymeren als auch den Füllstoffen besteht derzeit ein Bestreben, diese zunehmend aus nachwachsenden Rohstoffen zu gewinnen.

So werden neuerdings vermehrt organische Füllstoffe auf Basis von nachwachsenden Rohstoffen eingesetzt. Dabei handelt es sich beispielsweise um Nussschalen oder Olivenkerne, Fasern, Hülsen, Samen oder sonstige Pflanzenbestandteile, welche beispielsweise als Mehl oder als auf eine bestimmte Körnung gemahlene Partikel eingesetzt werden. Die eingesetzten Füllstoffe können zudem auf Recyclaten basieren. Diese können mineralisch sein aber auch organische Anteile enthalten.

Beispiele für Formmassen mit Polymeren beziehungsweise Bindemitteln auf Basis nachwachsender Rohstoffe sind ebenfalls bekannt. Beide Polymere a) und b) können zudem ganz oder teilweise auf Recyclaten oder Mischungen dieser basieren.

Da diese Vielzahl von unterschiedlichen Anwendungen auch unterschiedliche Anforderungen an die jeweiligen Verbundwerkstoffe bzw. Kompositmassen zu deren Herstellung stellt, ist es notwendig, den Füllstoff und die Kompositmassen für nahezu jede Anwendung individuell anzupassen. Ein für eine erste Anwendung geeigneter Verbundwerkstoff ist meist nicht optimal für eine andere Anwendung geeignet. Der Einsatz von nicht optimal für eine spezielle Anwendung ausgelegten Verbundwerkstoffen ist meist auch unwirtschaftlich, da sie oft kostenintensive Rohstoffen in einem erhöhten Mengenanteil enthalten, die für die vorgesehene Anwendung nicht oder zumindest nicht in dieser Menge notwendig wären.

Dementsprechend ist es technisch vorteilhaft und meist auch wirtschaftlich sinnvoll, für eine spezielle Anwendung auch die Füllstoffe und Kompositmassen entsprechend anzupassen. Dies ist jedoch im Stand der Technik mit einem enormen Entwicklungsaufwand verbunden. Der jeweilige Füllstoff und die jeweilige Kompositmasse muss dazu - um für eine spezielle Anwendungen geeignet sein zu können - entwickelt und getestet werden. Dazu wurden bislang eine Vielzahl von Füllstofflösungen und Kompositmassen im Labormaßstab hergestellt und deren Eigenschaften in teilweise aufwändigen Laboruntersuchungen gemessen. Füllstoffe und/oder Kompositmassen, die aufgrund ihrer Eigenschaften nicht zur Verwendung in einem Verfahren zur Umsetzung zu einem Werkstück geeignet sind (beispielsweise aufgrund deren rheologischen Verhalten) mussten bereits auf diese Entwicklungsstufe verworfen werden.

Noch langwieriger und mit noch mehr Ausschuss verbunden sind jedoch die Tests zur Umsetzung von im Labor plausiblen Rezeptkandidaten zu Gießwerkstücken. Die Umsetzung zu Gießwerkstücken ist insbesondere aufgrund der Zeiten, die für die Polymerisation und/oder Härtung des Bindemittels notwendig sind, sehr zeitintensiv. Darüber hinaus ist eine parallele Testung verschiedener Kompositmassenkandidaten oft aufgrund der zur Verfügung stehenden Anzahl von geeigneten Gießformen nur begrenzt möglich. Insbesondere bei der Testung auf eine Eignung zur Ausbildung komplexer geometrischer Formen (beispielsweise mit engen Krümmungsradien), ist somit nur eingeschränkt möglich. Schließlich ist der Materialaufwand und damit die Kosten für entsprechende Prozessprüfungen sehr hoch, da für jede Versuchsvariante zunächst ein konstanter Prozess eingestellt werden muss und die gebildeten Gießwerkstücke idR entsorgt werden müssen. Die negativen Aspekte im Zusammenhang mit Nachhaltigkeit kommen ergänzend dazu.

In der Vergangenheit wurden daher oftmals Untersuchungen durchgeführt, die lediglich die Auswirkungen der Veränderung einzelner Inhaltsstoffe oder deren Anteils auf verschiedene Parameter der Kompositmasse beziehungsweise des Gießwerkstücks untersuchten. Jedoch bleibt auch die Ermittlung des Effekts einzelner Bestandteile auf die Eigenschaften der Kompositmasse beziehungsweise der Gießform aus den oben genannten Gründen zeit- und kostenintensiv. Außerdem sind derartige Untersuchungen nicht sehr zuverlässig, da bei diesen Testreihen Synergieeffekte oder auch nachteilige Effekte zwischen verschiedenen Komponenten einer Kompositmasse beziehungsweise des Gießwerkstück eventuell nicht korrekt abgebildet werden. Sollten auch derartige Effekte berücksichtigt werden, wäre eine noch mehrfach größere Anzahl von Laboruntersuchungen notwendig, die das Kosten/Nutzen-Verhältnis in den meisten Fällen sprengen würde. Zudem würde eine solche Untersuchung in den meisten Fällen zu einer enormen Datenmenge führen, deren Auswertung ebenfalls einen enormen Zeitaufwand hat.

Bisher wurde daher versucht, einige wenige Bestandteile von Füllstoffen, Kompositmassen beziehungsweise Gießwerkstücken zu identifizieren und deren gegenseitige positive oder negative Beeinflussung innerhalb einer Kompositmasse oder eines Verbundwerkstoffs zu ermitteln. Diese Untersuchungen liefern jedoch kein zufriedenstellendes Ergebnis, da einerseits eine Beschränkung auf eine geringe Anzahl von Komponenten notwendig ist und andererseits dennoch eine große Datenmenge erzeugt wird und ausgewertet werden muss.

Der vorliegenden Erfindung liegt daher die Aufgabe zugrunde, die aus dem Stand der Technik bekannten Nachteile zu überwinden und ein Verfahren sowie eine Eigenschaftsermittlungsvorrichtung zur Ermittlung von Rezepturen für a) Füllstoffmischungen für b) eine Kompositmasse und c) zur Herstellung eines Gießwerkstücks bereitzustellen. Dieses sollte möglichst eine verlässliche und gleichwohl schnelle und möglichst wenig rechenintensive Möglichkeit der Vorhersage mindestens einer Eigenschaft einer Füllstofflösung und/oder Kompositmasse und/oder eines daraus herstellbaren Gießwerkstücks bieten.

Diese Aufgabe wird erfindungsgemäß durch die Gegenstände der unabhängigen Ansprüche gelöst. Vorteilhafte Ausführungsformen und Weiterbildungen der Erfindung sind Gegenstand der abhängigen Ansprüche.

Bei einem erfindungsgemäßen Verfahren zur, insbesondere prädiktiven, Erzeugung eines ersten Zusammensetzungsdatensatzes umfasst ein solcher Zusammensetzungsdatensatz Daten für die Rezeptierung einer ersten Füllstoffzusammensetzung und/oder einer ersten Kompositmasse für die Herstellung eines ersten Gießwerkstückes. Dieser erste Zusammensetzungsdatensatz umfasst:
- eine Information zu einer ersten Komponente und wenigstens einer weiteren, von der ersten Komponente verschiedenen zweite Komponente,
- eine Anteilsgröße der ersten Komponente und eine Anteilsgröße der zweiten Komponente in der ersten Füllstoffzusammensetzung und/oder der ersten Kompositmasse und/oder dem ersten Gießwerkstück, und
- eine Anteilsgröße der ersten Komponente und eine Anteilsgröße der zweiten Komponente in der ersten Kompositmasse und/oder dem ersten Gießwerkstück und.
- eine Information zu mindestens einer durch eine erste Analysegröße charakterisierbaren Eigenschaft der ersten r Kompositmasse und/oder des ersten Gießwerkstücks.

Das Verfahren umfasst die folgenden Schritte:
a) Erfassung eines zweiten Zusammensetzungsdatensatzes zu einer zweiten Füllstoffzusammensetzung und/oder Kompositmasse und/oder einem zweiten Gießwerkstück. Dabei umfasst der zweite Zusammensetzungsdatensatz wenigstens eine zweite Analysegröße und eine Anteilsgröße einer zweiten Komponente und bevorzugt einer Vielzahl von Anteilsgrößen von weiteren Komponenten.
b) Erfassung mindestens eines weiteren Zusammensetzungsdatensatzes zu mindestens einer weiteren Füllstoffzusammensetzung und/oder einer weiteren Kompositmasse und/oder einem weiteren Gießwerkstück. Dabei umfasst der mindestens eine weitere Zusammensetzungsdatensatz wenigstens eine weitere Analysegröße und eine weitere Anteilsgröße einer weiteren Komponente und bevorzugt eine Vielzahl von weiteren Anteilsgrößen von weiteren Komponenten. Außerdem ist dabei die zweite und/oder weitere Analysegröße jeweils charakteristisch ist für wenigstens eine Eigenschaft der/des durch den jeweiligen Zusammensetzungsdatensatz charakterisierten Füllstoffzusammensetzung und/oder Kompositmasse und/oder Gießwerkstücks.
   Weiterhin ist in einem solchen weiteren Zusammensetzungsdatensatz die zweite und/oder weitere Anteilsgröße jeweils charakteristisch für einen Mengenanteil der in der/dem durch den jeweiligen Zusammensetzungsdatensatz charakterisierten Füllstoffzusammensetzung und/oder Kompositmasse und/oder Gießwerkstück befindlichen ersten und/oder weiteren Komponente.
c) Computer-implementierte Ermittlung von wenigstens einem Wert des ersten Zusammensetzungsdatensatzes auf Grundlage des zweiten Zusammensetzungsdatensatzes und mindestens eines weiteren Zusammensetzungsdatensatzes. Dabei ist der ermittelte Wert ausgewählt aus einer Information aus einer Gruppe von Informationen
   - über das Vorhandensein oder Nicht-Vorhandensein einer ersten Komponente und/oder der wenigstens einen weiteren Komponente in der ersten Füllstoffzusammensetzung und/oder Kompositmasse und/oder dem ersten Gießwerkstück,
   - über eine Anteilsgröße der ersten und/oder weiteren Komponente in der ersten Füllstoffzusammensetzung und/oder der ersten Kompositmasse und/oder dem ersten Gießwerkstück, und
   - zu der mindestens einen durch die erste Analysegröße charakterisierbaren ersten Eigenschaft der ersten Füllstoffzusammensetzung und/oder der ersten Kompositmasse und/oder des ersten Gießwerkstück.

Als ein Gießwerkstück soll im Rahmen dieser Verbindung jeglicher Verbundwerkstoff verstanden werden, welcher aus einer Kompositmasse und einer Füllstoffzusammensetzung herstellbar ist. Dabei ist es unabhängig, ob das Gießwerkstück tatsächlich durch Gießen der Kompositmasse erzeugt wird oder durch ein anderes Verfahren. Denkbar wäre beispielsweise auch das Thermoformen, der 3D-Druck oder Spritzgießen.

Als eine Information zu einer (ersten) Komponente einer Füllstoffzusammensetzung und/oder einer Kompositmasse und/oder dem Gießwerkstück soll im Rahmen dieser Erfindung jegliche Information verstanden werden, die zu dieser Komponente verfügbar ist. diese Information kann beispielsweise durch einen Zahlenwert oder einen Binärcode verschlüsselt sein, welcher eineindeutig dieser Information zugeordnet werden kann. Der Mengenanteil dieser Komponente in der Füllstoffzusammensetzung und/oder der Kompositmasse und/oder dem Gießwerkstück ist jedoch bevorzugt nicht von einer solchen Information umfasst, da dieser Wert bevorzugt separat gehandhabt und in dem Zusammensetzungsdatensatz abgelegt ist.

Die Information zu einer ersten Komponente der ersten Füllstoffzusammensetzung und/oder der ersten Kompositmasse und/oder dem ersten Gießwerkstück ist im Rahmen dieser Erfindung oftmals mit dem Bezugszeichen "A₁" gekennzeichnet. Dabei soll der Buchstabe "A" die Substanz oder das Substanzgemisch symbolisieren, die in der ersten Füllstoffzusammensetzung und/oder der ersten Kompositmasse und/oder dem ersten Gießwerkstück enthalten ist. Der Index "₁" kann dabei eine Information zu einer besonderen Ausgestaltung oder Eigenschaft dieser Substanz oder das Substanzgemisch stehen, wie diese in der ersten Füllstoffzusammensetzung und/oder der ersten Kompositmasse und/oder dem ersten Gießwerkstück vorliegt. So kann der Index beispielsweise einen Hersteller, ein Herstellungsdatum, eine Chargennummer, eine Reinheit, eine Konzentration, eine bestimmte Verunreinigung, ein Lösungsmittel, ein Lösungsmittelanteil, eine Partikelgröße, eine Partikelgrößenverteilung, eine Molmassenverteilung, eine Struktur, einen Verzweigungsgrad, einen Schmelzpunkt, einen Glaspunkt und andere Eigenschaften der Substanz oder des Substanzgemisches oder Kombinationen von Eigenschaften symbolisieren. Das Bezugszeichen "A₁" ist dementsprechend in der Bedeutung zu verstehen, dass die Substanz "A" mit den speziellen Eigenschaften eingesetzt wird, wie sie für diese erste Komponente bekannt sind. Das Bezugszeichen "A₂" steht dementsprechend für dieselbe Substanz den Eigenschaften wie sie für diese zweite Komponente bekannt sind. Dabei kann es sein, dass zumindest einige Eigenschaften identisch sind. Unterscheiden sich die Komponenten "A₁" und "A₂" stark voneinander, wäre es möglich, dass diese Komponente anstatt dem Buchstaben "A" durch einen anderen Buchstaben, beispielsweise "B" symbolisiert wird. Dies wäre beispielsweise bei Copolymeren sinnvoll die zwar aus denselben Monomeren aufgebaut sind, diese jedoch in anderen Anteilen, anderen Abfolgen und/oder bei einem anderen Vernetzungsgrad enthalten.

Im Umkehrschluss können auch die erste Komponente "A₁" und die zweite Komponente "A₂" nicht nur die gleiche Substanz sein, sondern sich auch in ihren übrigen Eigenschaften nicht unterscheiden und somit identisch sein. Einziger Unterschied zwischen einer solchen ersten Komponente "A₁" und dieser zweiten Komponente "A₂" ist in einem solchen Fall dann, dass "A₁" Bestandteil der ersten Füllstoffzusammensetzung und/oder der ersten Kompositmasse und/oder des ersten Gießwerkstücks ist und "A₂" Bestandteil der zweiten Füllstoffzusammensetzung und/oder der zweiten Kompositmasse und/oder des zweiten Gießwerkstücks.

Eine Anteilsgröße einer Komponente in einer Füllstoffzusammensetzung und/oder Kompositmasse und/oder einem Gießwerkstück soll, sofern nichts anderes angegeben ist, als eine Information zu einem Gewichtsanteil der jeweiligen Komponente in einer Füllstoffzusammensetzung und/oder Kompositmasse und/oder einem Gießwerkstück verstanden werden. Dabei ist zu beachten, dass auch wenn ein Gießwerkstück aus einer bestimmten Kompositmasse bzw. einer Füllstoffzusammensetzung hergestellt wird, sich die Anteilsgröße für die jeweilige Komponente in der Kompositmasse und/oder dem Gießwerkstück unterscheiden kann. Ein solche Unterschied kann beispielsweise durch Anteilsveränderungen während der Polymerisation, Verdunstung von Lösungsmittel und/oder die Abspaltung von (beispielsweise gasförmigen) Reaktionsprodukten während der Umformung zu einem Gießwerkstück hervorgerufen werden. Das Bezugszeichen "N_{A2}" für eine Anteilsgröße soll dabei symbolisieren, dass dabei der Anteil "N" der Komponente "_{A2}" - also der Substanz A, wie sie in der zweiten Füllstoffzusammensetzung und/oder der zweiten Kompositmasse und/oder dem zweiten Gießwerkstück vorliegt - gemeint ist. Selbstverständlich ist es möglich, dass Anteilsgrößen für verschiedene Komponenten in einer oder verschiedenen Füllstoffzusammensetzungen und/oder Kompositmassen und/oder Gießwerkstücken einen identischen Wert annehmen.

Bevorzugt ist, dass in einem Zusammensetzungsdatensatz jeder Komponente (insbesondere jeweils genau) eine Anteilsgröße zugeordnet ist. Vorzugsweise bezieht sich folglich jede Anteilsgröße auf eine ihr zugeordnete (vorgegebene und/oder vorgebbare) Komponente der/des jeweiligen Füllstoffzusammensetzung und/oder Kompositmasse und/oder Gießwerkstücks, welche durch den jeweiligen Zusammensetzungsdatensatz charakterisiert ist.

Als eine Information zu mindestens einer durch eine Analysegröße charakterisierbaren Eigenschaft einer Füllstoffzusammensetzung und/oder einer Kompositmasse und/oder eines Gießwerkstücks soll ein Wert verstanden werden, der bei einer Analyse dieser Kompositmasse und/oder dieses Gießwerkstücks auf eine bestimmte Eigenschaft hin messbar oder erwartbar ist. So kann eine Eigenschaft einer Füllstoffzusammensetzung und/oder einer Kompositmasse und/oder eines Gießwerkstücks beispielsweise eine Farbe sein, die durch eine entsprechende Analysegröße, beispielsweise Werte eines Frequenzspektrums des sichtbaren Wellenlängenbereich, charakterisierbaren ist. Weitere Beispiele für mögliche Analysegrößen bzw. Eigenschaften sind Viskosität, Glanz, Farbverteilung, Rauhigkeit, Schallabsorption, Dichte, Dichteverteilung, Härte, E-Modul, Duktilität, (Schlag-) Zähigkeit, Bruchfestigkeit, Reißfestigkeit, Elastizität, Biegesteifigkeit, Temperaturbeständigkeit, Temperaturwechselbeständigkeit, Schmelzpunkt, Erweichungstemperatur, Zersetzungstemperatur, Viskosität, UV-Beständigkeit, Verschleißfestigkeit, Korrosionsbeständigkeit (beispielsweise gegenüber Wasser, feuchter Luft, Sauerstoff), Säurebeständigkeit bzw. Laugenbeständigkeit, Brennbarkeit und (Öko-) Toxizität.

Unter einer "Erfassung eines (zweiten, dritten oder weiteren) Zusammensetzungsdatensatzes" wird insbesondere ein Empfangen des über eine Kommunikationseinrichtung übermittelten Zusammensetzungsdatensatzes und/oder ein Abrufen des Zusammensetzungsdatensatzes von einer, insbesondere nichtflüchtigen, Speichereinrichtung (etwa einer externen Speichereinrichtung wie beispielsweise einem externen (Backend-)Server) durch eine, insbesondere Prozessorbasierte und bevorzugt nachfolgend näher beschriebene, Kompositmassenrezept-Ermittlungsvorrichtung verstanden, so dass (durch das Erfassen) bevorzugt ein Zusammensetzungsdatensatz (oder mehrere Zusammensetzungsdatensätze) zur Datenverarbeitung des Zusammensetzungsdatensatzes bzw. der Zusammensetzungsdatensätze bereitgestellt wird.

Daneben kann gemäß einer weiteren Ausführungsform unter "Erfassung eines (zweiten, dritten oder weiteren) Zusammensetzungsdatensatzes" zusätzlich oder alternativ auch ein Ermitteln und/oder Erzeugen des Zusammensetzungsdatensatzes zu verstehen sein. Dies könnte durch rechnerische Ermittlung, etwa durch Interpolation und/oder Extrapolation vorgegebener Datenwerte (etwa wenigstens einer oder mehrerer ähnlicher Füllstoffzusammensetzungen und/oder Kompositmassen und/oder Gießwerkstücke) und/oder durch experimentelles Erzeugen bzw. experimentelles Bestimmen und/oder experimentelles Messen (von Werten) des Zusammensetzungsdatensatzes erfolgen.

Wie oben dargelegt, enthält ein Zusammensetzungsdatensatz Daten zu Analysegröße/n, Anteilsgröße/n und Komponente/n. Ein im Zusammensetzungsdatensatz enthaltener Wert steht dabei vorzugsweise eindeutig, insbesondere eineindeutig für eine Analysegröße, eine Anteilsgröße oder eine Komponente. Der Begriff "Wert" kann als ein einzelner Eintrag in einem Datensatz und/oder ein einzelner Datenpunkt in einer Gruppe von Daten verstanden werden. Es kann sich bei einem solchen Wert um eine Zahl, eine Funktion, einen Buchstaben eine Buchstabenfolge oder Kombinationen davon handeln.

Im Folgenden wird nicht zwingend zwischen einem (beispielsweise als Binärcode angegebenen) Wert, der eine Analysegröße, eine Anteilsgröße oder eine Komponente in einem Zusammensetzungsdatensatz charakterisiert und dem tatsächlichen Wert der Analysegröße (beispielsweise Dichte 1,2 kg/l) oder Anteilsgröße (beispielsweise 5 Gewichts-%), beziehungsweise der tatsächlich in einer Füllstoffzusammensetzung und/oder Kompositmasse und/oder einem Gießwerkstücks enthaltenen Komponente (beispielsweise Quarz) unterschieden. Sofern der Unterschied nicht explizit beschrieben ist, soll im Rahmen dieser Beschreibung der Erfindung eine Offenbarung zu einem Wert eines Zusammensetzungsdatensatz, der eine Analysegröße, eine Anteilsgröße oder eine Komponente in einem Analysedatensatz charakterisiert, auch als Offenbarung für den tatsächlichen Wert der Analysegröße oder Anteilsgröße, beziehungsweise der tatsächlich in einer Füllstoffzusammensetzung und/oder einer Kompositmasse und/oder einem Gießwerkstück enthaltenen Komponente gelten. Analog soll auch eine Beschreibung für einen tatsächlichen Wert der Analysegröße oder Anteilsgröße, beziehungsweise der tatsächlich in einer Füllstoffzusammensetzung und/oder einer Kompositmasse und/oder einem Gießwerkstücks enthaltenen Komponente als Offenbarung für einen Wert in einem Zusammensetzungsdatensatz gelten, welcher diese Analysegröße, Anteilsgröße oder Komponente charakterisiert.

Analog soll im Rahmen dieser Beschreibung der Erfindung auch jede Beschreibung, die für einen Zusammensetzungsdatensatz gemacht ist als für die Füllstoffzusammensetzung und/oder die Kompositmasse und/oder das Gießwerkstück als offenbart gelten, die/das durch diesen Zusammensetzungsdatensatz charakterisiert ist, sofern dies nicht explizit ausgeschlossen ist. Umgekehrt soll auch jedoch Offenbarung, die für eine Füllstoffzusammensetzung und/oder eine Kompositmasse und/oder ein Gießwerkstück gegeben ist, als eine Offenbarung für einen Zusammensetzungsdatensatz gelten, der diese Füllstoffzusammensetzung und/oder diese Kompositmasse und/oder dieses Gießwerkstück charakterisiert.

Ebenso soll im Rahmen dieser Beschreibung der Erfindung auch jede Beschreibung, die für eine Eigenschaft einer Füllstoffzusammensetzung und/oder einer Kompositmasse und/oder eines Gießwerkstücks beschrieben ist, auch als für eine zugehörige Analysegröße offenbart gelten und umgekehrt, da vorzugsweise eine in einem Zusammensetzungsdatensatz enthaltene Analysegröße eindeutig, bevorzugt eineindeutig eine Eigenschaft einer Füllstoffzusammensetzung und/oder einer Kompositmasse und/oder eines Gießwerkstücks charakterisiert.

Durch das oben beschriebene Verfahren kann bevorzugt ermöglicht werden, dass ein erster Zusammensetzungsdatensatz ermittelt wird, der eine geeignete erste Füllstoffzusammensetzung und/oder eine Rezeptur einer ersten Kompositmasse abbildet, welche (gemeinsam oder unabhängig voneinander für die Herstellung eines ersten Gießwerkstückes geeignet sind. Dabei führt der Einsatz der ersten Füllstoffzusammensetzung und/oder der ersten Kompositmasse bevorzugt zu einem Gießwerkstück, das Eigenschaften aufweist, die zuvor als Zielparameter vorgegeben wurden. Bevorzugt kann die durch den ersten Zusammensetzungsdatensatz definierte Füllstoffzusammensetzung und/oder Kompositmasse zu einem Gießwerkstück mit diesen Eigenschaften umgesetzt werden. Dabei wäre es möglich, dass die so ermittelte erste Füllstoffzusammensetzung zunächst zu einer (bevorzugt durch einen separaten Zusammensetzungsdatensatz definierten) ersten Kompositmasse umgesetzt wird. Alternativ könnte jedoch auch nur die Zusammensetzung der ersten Füllstoffzusammensetzung durch das oben beschriebene Verfahren ermittelt werden und diese Füllstoffzusammensetzung in einer vorgegebenen (Standart-) Rezeptur für eine Kompositmasse eingesetzt werden. Dennoch führt bevorzugt auch bei dieser Variante der Einsatz der durch das oben beschriebene Verfahren ermittelten ersten Füllstoffzusammensetzung bzw. der durch den Zusammensetzungsdatensatz definierenden ersten Füllstoffzusammensetzung bei der Umsetzung zu einem Gießwerkstück zu einem Gießwerkstück mit den gewünschten Eigenschaften. Dementsprechend kann das oben beschriebene Verfahren einstufig oder mehrstufig sein.

Bevorzugt erfolgt die Computer-implementierte Ermittlung des wenigstens einen Werts des ersten Zusammensetzungsdatensatzes auf Grundlage von einer Vielzahl von zu verschiedenen Füllstoffzusammensetzung und/oder Kompositmassen und/oder Gießwerkstücken jeweils erfassten Zusammensetzungsdatensätzen. Bei dieser Verfahrensvariante ist es somit möglich, einen Wert, vorzugsweise eine Analysegröße des ersten Zusammensetzungsdatensatzes - und damit auch eine Eigenschaft der ersten Füllstoffzusammensetzung und/oder der ersten Kompositmasse und/oder des ersten Gießwerkstücks - zu ermitteln, ohne die ersten Füllstoffzusammensetzung und/oder ersten Kompositmasse und/oder das erste Gießwerkstücks tatsächlich auf diese Analysegröße und/oder Eigenschaft hin tatsächlich (physiko-chemisch) analysiert werden muss. Vielmehr ist es möglich, diese Analysegröße und/oder Eigenschaft (vorzugsweise prädiktiv) rechnerisch (vorzugsweise computergestützt) zu ermitteln. Als Basis für diese Berechnungen werden die Daten von Zusammensetzungsdatensätzen herangezogen, die beispielsweise in einer Datenbank für diesen Zweck vorgehalten werden.

Bevorzugt ist jeder Zusammensetzungsdatensatz (bevorzugt durch die Werte der Anteilsgrößen, welche den jeweiligen Komponenten zugeordnet sind) jeweils charakteristisch für eine Füllstoffzusammensetzung und/oder eine Kompositmasse und/oder ein Gießwerkstück, so dass insbesondere bevorzugt die verschiedenen Füllstoffzusammensetzungen und/oder Kompositmassen und/oder das erste Gießwerkstücke auf Grundlage der jeweiligen Zusammensetzungsdatensätze voneinander (bevorzugt eineindeutig) unterscheidbar sind. Denkbar wäre aber auch, dass zwei (oder mehr) Füllstoffzusammensetzungen und/oder Kompositmassen und/oder Gießwerkstücke derart ähnlich in ihrer Zusammensetzung/Rezeptur sind, dass ihre jeweiligen Zusammensetzungsdatensätze (im Wesentlichen bzw. ggf. im Rahmen einer (Mess-)Toleranz) gleich sind.

Bevorzugt umfasst die erste Füllstoffzusammensetzung und/oder erste Kompositmasse und/oder das erste Gießwerkstück eine Vielzahl von Komponenten, wobei beispielsweise mehr als zwei, bevorzugt mehr als drei, bevorzugt mehr als fünf, bevorzugt mehr als acht, bevorzugt mehr als zehn und besonders bevorzugt mehr als 15 Komponenten in der ersten Füllstoffzusammensetzung und/oder der ersten Kompositmasse und/oder dem ersten Gießwerkstück enthalten sein können bzw. zu der ersten Füllstoffzusammensetzung und/oder der ersten Kompositmasse und/oder dem ersten Gießwerkstück umgesetzt werden können. Unter den Komponenten sind insbesondere Substanzen oder Abmischungen zu verstehen, die aktiv zusammengeführt werden (können), um die erste Füllstoffzusammensetzung und/oder die erste Kompositmasse und/oder das erste Gießwerkstück zu erhalten. Eventuell in einer oder mehreren der Komponenten enthaltenen Verunreinigungen bleiben bei dieser Betrachtung unberücksichtigt. Werden auch eventuell enthaltene Verunreinigungen als eigenständige Komponenten betrachtet, kann deren Anzahl in der ersten Kompositmasse und/oder dem ersten Gießwerkstück auch deutlich über 50 oder sogar > 100 liegen, da oftmals die B-Komponenten lediglich in technischer Reinheit und/oder beispielsweise einer Reinheit ≥ 99 Gewichtsprozent eingesetzt werden.

Die in dem Zusammensetzungsdatensatz oder den Zusammensetzungsdatensätzen enthaltenen Daten zu Analysegröße/n, Anteilsgröße/n und Komponente/n können (jeweils, bevorzugt unabhängig voneinander) rechnerisch ermittelt sein, oder durch eine (physiko-chemische) Analyse ermittelt sein. Vorzugsweise ist jedoch zumindest ein überwiegender Teil, vorzugsweise ≥ 75 %, bevorzugt ≥ 90 %, weiter bevorzugt ≥ 95 %, meist bevorzugt ≥ 99 % der in dem Zusammensetzungsdatensatz oder den Zusammensetzungsdatensätzen enthaltenen Daten durch eine (physiko-chemische) Analyse ermittelt. Ein hoher Anteil an durch eine (physiko-chemische) Analyse ermittelten Daten in dem Zusammensetzungsdatensatz oder den Zusammensetzungsdatensätzen hat den Vorteil, dass diese üblicherweise weniger fehlerbehaftet sind als rechnerisch ermittelte Daten, da bei rechnerisch ermittelten Daten mögliche Fehler in den Daten, die die Berechnungsgrundlage bilden, mitgeschleift oder sogar vergrößert werden können.

Vorzugsweise wird der zweite Zusammensetzungsdatensatz durch eine Analyse, vorzugsweise eine Vollanalyse, der zweiten Füllstoffzusammensetzung und/oder der zweiten Kompositmasse und/oder einem zweiten Gießwerkstück erhalten und/oder der mindestens eine weitere Zusammensetzungsdatensatz durch eine Analyse, vorzugsweise eine Vollanalyse, der mindestens einen weiteren Kompositmasse erhalten. Eine Analyse kann dabei darauf beschränkt sein, einen oder mehrere der in dem Zusammensetzungsdatensatz enthaltenen Daten zu ermitteln. Alternativ zu einer Analyse aller Daten des Datensatzes ein Wert oder mehrere Daten, insbesondere eine Anteilsgröße oder mehrere Anteilsgrößen, auch einer Rezeptur oder Formulierungsanweisung entnommen oder daraus berechnet werden. Insbesondere ist dementsprechend bevorzugt, dass eine Analysegröße oder mehre Analysegrößen durch eine Analyse der jeweiligen Eigenschaft der jeweiligen Füllstoffzusammensetzung und/oder der jeweiligen Kompositmasse und/oder dem jeweiligen Gießwerkstück ermittelt wird/werden und/oder die Anteilsgrößen der ersten, zweiten und/oder weiteren Komponente einer Rezeptur oder Formulierungsanweisung entnommen oder daraus berechnet werden. Dadurch kann der Analyseaufwand reduziert werden.

Eine Ermittlung einer Eigenschaft einer Füllstoffzusammensetzung und/oder einer Kompositmasse und/oder eines Gießwerkstücks auf Basis von Daten, die bereits bei der Produktion dieser Füllstoffzusammensetzung und/oder dieser Kompositmasse und/oder dieses Gießwerkstücks bekannt sind (nämlich insbesondere die eingesetzten Komponenten und deren jeweilige Anteilsgrößen), und die gegebenenfalls im Verlauf des Transports und/oder Lagerns und/oder der weiteren Umsetzung zu einem Gießwerkstück erneut überprüft werden, ist dabei mit deutlich weniger Aufwand verbunden (im Vergleich zu einer experimentellen Messung der Eigenschaften der Füllstoffzusammensetzung und/oder Kompositmasse und/oder des Gießwerkstücks).

Ein Zusammensetzungsdatensatz umfasst vorzugsweise jeweils Paare von Daten (Im Folgenden auch als Deskriptoren bezeichnet), die jeweils charakteristisch sind für eine Komponente und deren Anteilsgröße in der/dem durch diesen Zusammensetzungsdatensatz charakterisierten Füllstoffzusammensetzung und/oder Kompositmasse und/oder Gießwerkstück. Außerdem umfasst ein Zusammensetzungsdatensatz vorzugsweise mindestens einen Wert, der für eine Analysegröße charakteristisch ist. Bevorzugt ist jedoch, dass ein Zusammensetzungsdatensatz eine Vielzahl von für Analysegrößen charakteristische Werte enthält, wobei jeder Wert vorzugsweise für (bevorzugt genau) eine Analysegröße charakteristisch ist.

Insbesondere ist bevorzugt, dass ein Zusammensetzungsdatensatz (bevorzugt jeder oder zumindest nahezu jeder Zusammensetzungsdatensatz) mehrere der oben genannten Paare von Daten umfasst. Es hat sich gezeigt, dass eine Füllstoffzusammensetzung und/oder eine Kompositmasse und/oder ein Gießwerkstück ausreichend genau charakterisiert sein kann, sofern mindestens zwei derartige Paare definiert sind. Eine so geringe Anzahl von Daten ist insbesondere dann ausreichend, wenn eine der durch ein solches Datenpaar charakterisierten Komponenten eine Abmischung mehrerer Substanzen ist. Vorzugsweise umfasst ein Zusammensetzungsdatensatz jedoch mehrere der oben genannten Paare von Daten, bevorzugt mindestens 4, weiter bevorzugt mindestens 6 weiter bevorzugt mindestens 8 und meist bevorzugt mindestens 10 der oben genannten Paare von Daten.

Vorzugsweise umfasst ein Zusammensetzungsdatensatz Paare von Daten, die für Komponenten und deren Anteilsgrößen charakteristisch sind, die in einem so großen Anteil in der Füllstoffzusammensetzung und/oder der Kompositmasse und/oder dem Gießwerkstück enthalten sind, dass sie einen signifikanten Einfluss auf mindestens eine Eigenschaft der jeweiligen Füllstoffzusammensetzung und/oder der Kompositmasse und/oder des jeweiligen Gießwerkstücks, insbesondere auf eine oder die eine durch die Analysegröße abgebildete Eigenschaft haben. Da ein großer Anteil der Füllstoffzusammensetzung und/oder der Kompositmasse und/oder des Gießwerkstücks durch ein Polymer und/oder einen Füllstoff gebildet sein kann, kann der Anteil einer (oder mehrerer) anderen Komponente(n) sehr klein sein, und die jeweilige Komponente kann dennoch einen merklichen Einfluss auf die gesamte Füllstoffzusammensetzung und/oder die Kompositmasse und/oder das Gießwerkstück haben. Dementsprechend ist vorzugsweise keine untere Grenze der Anteilsgröße definiert. Es hat sich jedoch gezeigt, dass lediglich als Verunreinigung anzusehende Anteilsgrößen vernachlässigbar sein können. Vorzugsweise umfasst ein Zusammensetzungsdatensatz daher Paare von Daten, bei denen die jeweilige Anteilsgröße einen (Gewichts-) Anteil von mindestens 1:1.000.000 (bezogen auf das Gesamtgewicht aller Komponenten in einer Füllstoffzusammensetzung und/oder einer Kompositmasse und/oder dem Gießwerkstück), bevorzugt von mindestens 1:100.000, meist bevorzugt 1:10.000 aufweist.

Durch eine solche Beschränkung auf für mindestens eine Analysegröße relevante Paare von Daten in einem Zusammensetzungsdatensatz kann die Datenmenge vergleichsweise gering gehalten werden. So ist beispielsweise eine Beschränkung auf ≤ 50, bevorzugt ≤ 40, weiter bevorzugt ≤ 30, mehr bevorzugt ≤ 25 und meist bevorzugt ≤ 20 Paaren von Daten pro Analysedatensatz möglich.

Ebenso ist es vorteilhaft, die Anzahl der möglichen Komponenten zu beschränken. Dies ist nicht nur im Hinblick auf die Größe der zu handhabende Datenmenge vorteilhaft, sondern reduziert insbesondere auch die notwendige Rechenleistung. Vorteilhaft ist daher die Anzahl erlaubter Komponenten ≤ 1000, bevorzugt ≤ 750, weiter bevorzugt ≤ 500, mehr bevorzugt ≤ 400 und meist bevorzugt ≤ 350. Dabei werden vorzugsweise diejenigen Komponenten ausgewählt, die in marktbekannten Füllstoffzusammensetzungen und/oder Kompositmassen und/oder Gießwerkstücken enthalten sind und/oder von denen man weiß oder zumindest vermutet, dass sie einen signifikanten Einfluss auf mindestens eine Analysegröße haben.

Bevorzugt weist der (insbesondere jeder) Zusammensetzungsdatensatz mindestens eine Analysegröße auf. So wäre beispielsweise denkbar, dass ausschließlich die Viskosität der Kompositmasse als einzige Analysegröße verwendet würde. Diese Information könnte beispielsweise zum Bewerten verschiedener Füllstoffzusammensetzungen und/oder Kompositmassen zur Erzeugung von Gießwerkstücken mit besonders engen Kurvenradien und/oder komplexen Geometrien herangezogen werden.

Bevorzugt ist jedoch, dass ein (insbesondere jeder) Zusammensetzungsdatensatz nicht nur eine einzige Analysegröße sondern mehrere Analysegrößen umfasst. Es ist bei Füllstoffzusammensetzungen und/oder Kompositmassen und/oder Gießwerkstücken üblich, diese durch mehrere (für Anwender relevante) Daten zu charakterisieren. Diese Daten werden für eine Füllstoffzusammensetzung und/oder Kompositmasse und/oder Gießwerkstück daher meist ohnehin ermittelt, so dass sie zur Verfügung stehen und vergleichsweise einfach in einen Zusammensetzungsdatensatz eingefügt werden können. Die Angabe mehrerer Analysegrößen in einem Zusammensetzungsdatensatz ist vorteilhaft, da so bei der Ermittlung der (physiko-chemischen) Eigenschaft der Füllstoffzusammensetzung und/oder Kompositmasse und/oder des Gießwerkstücks nicht nur diese, sondern auch andere Eigenschaften ermittelbar sind. Ein Fachmann (oder ein Computersystem) kann somit unmittelbar eine Plausibilitätsprüfung durchführen und Ermittlungsresultate ausschließen oder verwerfen, für die mindestens eine Eigenschaft beziehungsweise eine Analysegröße außerhalb eines zulässigen Bereichs liegt. Bevorzugt umfasst daher ein (insbesondere jeder) Zusammensetzungsdatensatz mindestens 3, ≥ 5 Analysegrößen, bevorzugt ≥ 8, weiter bevorzugt ≥ 12, mehr bevorzugt ≥ 15, insbesondere bevorzugt ≥ 20, meist bevorzugt ≥ 25 Analysegrößen.

Ergänzend oder alternativ dazu weist ein (insbesondere jeder) Zusammensetzungsdatensatz vorzugsweise ≤ 50, bevorzugt ≤ 30, weiter bevorzugt ≤ 25, meist bevorzugt ≤ 20 verschiedene Analysegrößen auf. Grundsätzlich wäre es auch möglich, Zusammensetzungsdatensätze mit einer größeren Anzahl verschiedener Analysegrößen zu nutzen, jedoch wird sich im Hinblick auf die Menge der zu speichernden Daten sowie der geforderten Rechenleistung vorzugsweise auf die oben genannte Anzahl der relevantesten Analysegrößen beschränkt. Für die meisten kommerziell erhältlichen Füllstoffzusammensetzung und/oder Kompositmassen und/oder Gießwerkstücke wird in den Produktinformationen auch lediglich eine deutlich geringere Anzahl relevanter Kenndaten zur Charakterisierung der Produkteigenschaften angegeben.

Die vorgenannten maximalen Anzahlen für die jeweiligen Analysegrößen bieten jeweils (einzeln oder in Kombination) den Vorteil, dass zum einen typische Daten, welche bei der Herstellung der Füllstoffzusammensetzung und/oder Kompositmassen und/oder des Gießwerkstücken üblicherweise bestimmt werden, verwendet werden können und keine weiteren Messdaten zur Erfassung des Zusammensetzungsdatensatzes erzeugt bzw. bestimmt werden müssen und gleichzeitig eine möglichst weitgehende Reduktion der Größe der Zusammensetzungsdatensätze vorgenommen werden kann.

Es hat sich gezeigt, dass eine Datenbank eine Mindestanzahl von Zusammensetzungsdatensätzen umfassen sollte, um eine zuverlässige Ermittlung einer Eigenschaft der Kompositmasse und/oder des Gießwerkstücks zu ermöglichen. Daher sollte eine Datenbank ≥ 50, bevorzugt ≥ 100, weiter bevorzugt ≥ 150, mehr bevorzugt ≥ 175, meist bevorzugt ≥ 200 Zusammensetzungsdatensätze umfassen. Ermittlungen einer Eigenschaft der Kompositmasse und/oder des Gießwerkstücks können mit einer Datenbank dieser Größe mit akzeptabler Fehlertoleranz ermittelt werden. Denkbar und in vielen Fällen bevorzugt sind jedoch größere Datenbanken mit beispielsweise ≥ 500, bevorzugt ≥ 1000, weiter bevorzugt ≥ 1500, mehr bevorzugt ≥ 2000, meist bevorzugt ≥ 5000 Zusammensetzungsdatensätzen. Auch wenn bei diesen Datenbanken die zu handhabende Datenmenge ansteigt, hat sich dies in einigen Fällen als vorteilhaft erwiesen, da auf Basis einer derartigen Datenbank die Eigenschaft(en) der Kompositmasse und/oder des Gießwerkstücks mit höherer Genauigkeit und Robustheit ermittelbar ist/sind.

Vorzugsweise werden mehr als zwei Zusammensetzungsdatensätzen, beispielsweise ≥ 5 Zusammensetzungsdatensätzen vorzugsweise ≥ 10 Zusammensetzungsdatensätzen, weiter bevorzugt ≥ 20 Zusammensetzungsdatensätzen und meist bevorzugt ≥ 50 Zusammensetzungsdatensätzen herangezogen, um während des Schritts der computer-implementierten Ermittlung die wenigstens eine Information aus der oben genannten Gruppe zu ermitteln. Es hat sich gezeigt, dass ab einer solchen Anzahl von Zusammensetzungsdatensätzen eine sinnvolle und meist zutreffende Ermittlung möglich wird. Beispielsweise kann die (physiko-chemischen) Eigenschaft der ersten Füllstoffzusammensetzung und/oder der ersten Kompositmasse und/oder des ersten Gießwerkstücks bzw. die dafür charakteristische Analysegröße mit ausreichender Robustheit ermittelt werden.

Bevorzugt wird bei der Durchführung des Verfahrens mindestens eine erste Analysegröße, welche die erste Eigenschaft charakterisiert für die erste Füllstoffzusammensetzung und/oder der ersten Kompositmasse und/oder das erste Gießwerkstück vorgegeben und während der computer-implementierten Ermittlung des wenigstens einen Wertes des ersten Zusammensetzungsdatensatzes
a) das Vorhandensein oder Nicht-Vorhandensein einer ersten Komponente und/oder der wenigstens einen weiteren Komponente in der ersten Kompositmasse und/oder dem ersten Gießwerkstück, und
b) eine Anteilsgröße der ersten und/oder weiteren Komponente in der ersten Kompositmasse und/oder dem ersten Gießwerkstück
ermittelt. Durch diese Verfahrensvariante wird ermöglicht, dass ein Anwender Soll-Werte für eine oder mehrere ausgewählte Analysegrößen vorgibt und während des Verfahrens mögliche Zusammensetzungsdatensätze ermittelt werden, die mit hoher Wahrscheinlichkeit zur Herstellung von Füllstoffzusammensetzungen und/oder Kompositmassen und/oder Gießwerkstücken genutzt werden können, die die gewünschten, mit den Analysegrößen korrelierenden Eigenschaften aufweisen. So wäre es beispielsweise für Küchenspülen möglich, hohe Werte für die bei dieser Anwendung besonders relevanten Eigenschaften der Temperaturwechselbeständigkeit, Hitzebeständigkeit, Wasserbeständigkeit, Säurebeständigkeit, Laugenbeständigkeit, Duktilität und (Schlag-) Zähigkeit vorzugeben. Die dann bei der Durchführung der Verfahrensvariante erhaltenen Zusammensetzungsdatensätze würden bei der Umsetzung zu einem Gießwerkstück ermöglichen, Küchenspülen zu erhalten, die sich durch ihre Materialeigenschaft besonders für die Anwendung unter den in diesem Bereich besonderen Bedingungen geeignet sind.

Wie aus dem oben genannten Beispiel hervorgeht, ist es bevorzugt, dass ein Zusammensetzungsdatensatz mehrere Analysegrößen umfasst, um einem Nutzer beispielsweise bei der oben beschriebenen Anwendung die Möglichkeit zu geben, mehrere Analysegrößen bzw. dazu korrelierende Eigenschaften der Füllstoffzusammensetzung und/oder der Kompositmassen und/oder Gießwerkstücke vorzugeben.

Bevorzugt ist eine Verfahrensvariante, bei der ergänzend oder alternativ zu einer oder mehreren der oben dargelegten Varianten auf Grundlage eines Soll-Wertes wenigstens einer und bevorzugt einer Vielzahl von Eigenschaft(-en) der ersten Füllstoffzusammensetzung und/oder der ersten Kompositmasse und/oder des ersten Gießwerkstücks eine der ersten Füllstoffzusammensetzung und/oder der ersten Kompositmasse und/oder dem ersten Gießwerkstück zuzuführende erste Komponente und deren Anteilsgröße ermittelt wird und/oder eine der ersten Füllstoffzusammensetzung und/oder der ersten Kompositmasse und/oder dem ersten Gießwerkstück zuzuführende weitere Komponente und deren jeweilige Anteilsgröße ermittelt wird. Wird also beispielsweise eine bestimmte Eigenschaft, beispielsweise eine hohe Chemikalienbeständigkeit, vorgegeben, kann durch diese Verfahrensvariante ermittelt werden, welche erste Komponente und/oder weitere Komponente einer Füllstoffzusammensetzung und/oder einer Kompositmasse und/oder einem Gießwerkstück in welcher jeweiligen Anteilsgröße zugeführt werden sollte, um zu gewährleisten, dass die Füllstoffzusammensetzung und/oder die Kompositmasse und/oder das Gießwerkstück tatsächlich den Soll-Wert, nämlich im Beispiel eine hohe Chemikalienbeständigkeit, aufweist.

Bevorzugt wird der erste Zusammensetzungsdatensatz derart ermittelt, dass zu der wenigstens einen (physiko-chemischen) Eigenschaft, im Folgenden auch als Soll-Eigenschaft bezeichnet, und bevorzugt zu jeder (vorgegebenen) Eigenschaft eine erste Komponente sowie deren Anteilsgröße und wenigstens eine weitere, von der ersten Komponente verschiedene Komponente und deren Anteilsgröße ermittelt wird. Die Ermittlung der ersten Komponente sowie deren Anteilsgröße als auch der wenigstens einen weiteren Komponente und deren Anteilsgröße erfolgt wie oben dargelegt vorzugsweise auf Grundlage eines weiteren Zusammensetzungsdatensatzes und mindestens einem weiteren Zusammensetzungsdatensatz.

Bevorzugt wird für die (physiko-chemische) Eigenschaft einer ersten Kompositmasse und/oder eines ersten Gießwerkstücks die Soll-Eigenschaft beziehungsweise eine dazu korrelierende Analysegröße basierend auf einem Einfluss der Anteilsgröße der ersten Komponente sowie einem Einfluss der Anteilsgröße, der wenigstens einen weiteren Komponente auf die Soll-Eigenschaft bzw. die Analysegröße berechnet. Bevorzugt wird dazu einer, bevorzugt mehreren, meist bevorzugt jeder Komponente der Füllstoffzusammensetzung und/oder der Kompositmasse und/oder des Gießwerkstücks ein Gewichtungswert zugeordnet, welcher zu einem Einfluss der jeweiligen Anteilsgröße der jeweiligen Komponente auf eine Veränderung der Soll-Eigenschaft korreliert. So könnte beispielsweise einem Lösungsmittel als Komponente einer Kompositmasse ein hoher Gewichtungswert für die Soll-Eigenschaft der Viskosität zugeordnet werden, jedoch ein niedriger Gewichtungswert für die Soll-Eigenschaft einer Farbe der Kompositmasse und/oder eines daraus herstellbaren Gießwerkstücks.

Vorzugsweise wird für jede (vorgegebene) Eigenschaft (bzw. der diese charakterisierenden Analysegröße) einer Füllstoffzusammensetzung und/oder einer Kompositmasse und/oder eines Gießwerkstücks separat eine Anteilsgröße sowohl der ersten Komponente als auch der wenigstens einen weiteren Komponente bestimmt bzw. berechnet.

Bevorzugt umfasst mindestens der zweite Zusammensetzungsdatensatz und mindestens einer der weiteren Zusammensetzungsdatensätze einen Wert (eine Analysegröße) der für die (physiko-chemische) (Soll-) Eigenschaft der/des durch den jeweiligen Zusammensetzungsdatensatz charakterisierten zweiten oder weiteren Füllstoffzusammensetzung und/oder Kompositmasse und/oder Gießwerkstücks charakteristisch ist (jedoch nicht der ersten Kompositmasse und/oder des ersten Gießwerkstücks). Vorzugsweise umfasst eine Vielzahl von Zusammensetzungsdatensätzen einen für diese (Soll-) Eigenschaft charakteristischen Wert (bzw. eine entsprechende Analysegröße). Dadurch wird ermöglicht, dass jeder der Komponenten der zweiten oder weiteren Füllstoffzusammensetzung und/oder Kompositmasse und/oder Gießwerkstücks jeweils ein Gewichtungswert zugeordnet werden kann, welcher den Einfluss der jeweiligen Komponente auf die (Soll-) Eigenschaft abbildet.

Die Verwendung derartiger Zusammensetzungsdatensätze und eine Ermittlung von Gewichtungswerten für die jeweiligen Komponenten, bietet den Vorteil, dass die (hierfür) zu übermittelnde Datenmenge nicht so groß (wie im Stand der Technik bzw. bei Vollanalysen) ist und (wie nachfolgend erläutert) eine viel kleinere Anzahl an Datensätzen erforderlich ist, um ein robustes Ergebnis zu erhalten. Die Berechnung bzw. Ermittlung der physiko-chemischen (Soll-) Eigenschaft ist sehr schnell möglich und erfordert keine großen Versuchsreihen mit einer Vielzahl von verschiedenen Formulierungen zur Bereitstellung von Füllstoffzusammensetzungen und/oder Kompositmassen und/oder Gießwerkstücken für eine anschließende (physiko-chemische) Analyse.

In einer bevorzugten Verfahrensvariante erfolgt die computer-implementierte Ermittlung von wenigstens einer für die Eigenschaft der ersten Füllstoffzusammensetzung und/oder der ersten Kompositmasse und/oder dem ersten Gießwerkstück charakteristischen (physiko-chemischen) Eigenschaftsgröße rein computer-implementiert. Dazu wird wie oben beschrieben vorzugsweise der Zusammensetzungsdatensatz zu der zweiten Füllstoffzusammensetzung und/oder einer zweiten Kompositmasse und/oder dem zweiten Gießwerkstück und der mindestens eine weitere Zusammensetzungsdatensatz zu der mindestens einen weiteren Füllstoffzusammensetzung und/oder mindestens einer weiteren Kompositmasse und/oder dem mindestens einen weiteren Gießwerkstück zugrunde gelegt.

Bevorzugt erfolgt somit das Ermitteln der wenigstens einen Eigenschaftsgröße in einem Computer-implementierten Verfahrensschritt. Bevorzugt erfolgt das Ermitteln der wenigstens einen Eigenschaftsgröße mittels einer bzw. der (insbesondere nachfolgend beschriebenen), insbesondere Prozessor-basierten, Kompositmassenrezept-Ermittlungsvorrichtung.

Vorzugsweise umfasst der erste und/oder der zweite und/oder der weitere Zusammensetzungsdatensatz (insbesondere bevorzugt jeder Zusammensetzungsdatensatz) wenigstens eine Analysegröße und bevorzugt eine Vielzahl von Analysegrößen, beispielsweise ≥ 5 Analysegrößen, vorzugsweise ≥ 8 Analysegrößen, weiter bevorzugt ≥ 10 Analysegrößen und meist bevorzugt ≥ 15 Analysegrößen. Bevorzugt ist jede der Analysegrößen für eine Eigenschaft der/des durch den jeweiligen Zusammensetzungsdatensatz charakterisierten Füllstoffzusammensetzung und/oder Kompositmasse und/oder Gießwerkstücks charakteristisch. Eine Analysegröße kann mit anderen Worten als Identifikator (nachfolgend auch als "Identifier" bezeichnet) einer Eigenschaft einer Füllstoffzusammensetzung und/oder Kompositmasse und/oder eines Gießwerkstücks dienen, welche insbesondere betrachtet werden soll.

Vorzugsweise ist die Eigenschaft einer Füllstoffzusammensetzung und/oder einer Kompositmasse und/oder eines Gießwerkstücks, die wie oben dargelegt bevorzugt durch die Analysegröße charakterisiert ist, ausgewählt aus einer Gruppe, die Farbe, einen Farbwert, Farbverteilung, Gelbwert, Weißgrad, Glanz, Rauhigkeit, Schallabsorption, Dichte, Dichteverteilung, Schüttdichte, Härte, Partikelgrößenverteilung, Abrasionswiderstand, Nassabriebbeständigkeit, Atmungsaktivät, E-Modul, Duktilität, (Schlag-) Zähigkeit, Bruchfestigkeit, Reißfestigkeit, Elastizität, Biegesteifigkeit, Temperaturbeständigkeit, Temperaturwechselbeständigkeit, Schmelzpunkt, Gelzeit, Reaktivität, Erweichungstemperatur, Zersetzungstemperatur, Brennbarkeit, Feuerfestigkeit, Viskosität, UV-Beständigkeit, Verschleißfestigkeit, Korrosionsbeständigkeit (beispielsweise gegenüber Wasser, feuchter Luft, Sauerstoff), Säurebeständigkeit, Laugenbeständigkeit, pH-Wert, eine (Öko-) Toxizität, Schadstoffwert, Gesundheitsgefährdungsrisiko, Entsorgungsmöglichkeit, Feststoffanteil, Lösungsmittelanteil, Wasserlöslichkeit, Lagerfähigkeit und Diffusionseigenschaft umfasst. Diese Eigenschaften können dazu dienen, Füllstoffzusammensetzungen und/oder Kompositmassen und/oder eines Gießwerkstücke voneinander zu unterscheiden und deren Eignung für bestimmte Anwendungsfälle zu bewerten.

Vorzugsweise enthält jeder Zusammensetzungsdatensatz) für (zumindest nahezu) jede (oder jede bewusst zugesetzte) in der durch diesen Zusammensetzungsdatensatz charakterisierten Füllstoffzusammensetzung und/oder Kompositmasse und/oder Gießwerkstück enthaltene Komponente eine Anteilsgröße, welche für den Anteil dieser Komponente in der jeweiligen Füllstoffzusammensetzung und/oder Kompositmasse und/oder dem jeweiligen Gießwerkstück charakteristisch ist.

So könnte beispielsweise ein Zusammensetzungsdatensatz, welcher eine Füllstoffzusammensetzung und/oder Kompositmasse für die Herstellung eins Gießwerkstücks mit besonders harter Oberfläche charakterisiert, zumindest eine Anteilsgröße enthalten, welche für einen Füllstoff mit besonders hoher Härte nach Mohs charakteristisch ist. Daneben könnte dieser Zusammensetzungsdatensatz beispielsweise auch eine oder mehrere weitere Anteilsgröße/n umfassen, welche/r beispielsweise für einen Anteil eines Lösungsmittels, eines Bindemittels, eines Füllstoffs, eines Polymers und/oder eines Konservierungsmittels charakteristisch ist/sind. Eine Analysegröße könnte in einem solchen beispielhaften Zusammensetzungsdatensatz beispielsweise eine Kratzfestigkeit, eine Härte, eine Dichte, eine Viskosität und/oder eine Lagerbeständigkeit sein.

Vorzugsweise handelt es sich bei der ersten Komponente und der zweiten Komponente, vorzugsweise auch der mindestens einen weiteren Komponente, jeweils um einen Inhaltsstoff der Füllstoffzusammensetzung und/oder der Kompositmasse und/oder dem Gießwerkstück, vorzugsweise um ein Additiv. Ein Lösungsmittel kann jedoch auch als eine erste Komponente, zweite Komponente oder weitere Komponente einer Kompositmasse und/oder einem Gießwerkstück betrachtet werden. Insbesondere bei Kompositmassen ist das Lösungsmittel und dessen Anteil von großer Bedeutung, insbesondere im Hinblick auf deren Viskosität. Aber auch bei Gießwerkstücken kann der (Rest-) Lösungsmittelanteil relevant sein, insbesondere während des Prozesses der Formgebung und/oder Aushärtung des Gießwerkstücks, da der Lösungsmittelanteil beispielsweise die bis zur Entformung notwendige Zeit maßgeblich mitbestimmt.

Bevorzugt ist mindestens eine der ersten Komponente oder der wenigstens einen weiteren Komponente und eine andere der ersten Komponente oder der wenigstens einen weiteren Komponente der Füllstoffzusammensetzung und/oder der Kompositmasse und/oder des Gießwerkstücks unabhängig voneinander ausgewählt aus einer Gruppe, die ein Rheologieadditiv, ein Pigment, einen Farbstoff, eine Farbpaste, ein Effektpigment, einen Emulgator, ein Biozid, einen Härter, einen Beschleuniger, einen Inhibitor, einen Füllstoff, ein Lösungsmittel, ein Polymer, ein Monomer, einen Polymerisationsstarter, ein Organosilan, einen Quervernetzer, einen Entschäumer, ein Benetzungsadditiv, ein Quarz- und/oder Quarzfolgeprodukt, eine Ca- und/oder Mg-haltige Komponente, eine Al-haltige Komponente, ein Konservierungsmittel, insbesondere ein Film- und/oder Topfkonservierungsmittel, einen anorganischen und/oder organischen Füllstoff umfasst, der sowohl mineralisch, amorph, oder biogen vorliegen kann. Es werden Füllstoffe bevorzugt, die aus der Gruppe der Quarze, Cristobalite, Feldspäte, Calcite, Dolomite, Wollastonite stammen und alleine vorliegen können oder in Mischung in jedem Verhältnis. In dieser Ausführungsform sind somit mindestens zwei Komponenten unabhängig voneinander aus der oben genannten Gruppe ausgewählt.

Insbesondere ist bevorzugt, dass zusätzlich zu den oben genannten Komponenten mindestens eine, bevorzugt mindestens 2, weiter bevorzugt mindestens 4, mehr bevorzugt mindestens 6, noch mehr bevorzugt mindestens 8, insbesondere bevorzugt mindestens 10 weitere der ersten Komponente oder der wenigstens eine weiteren Komponente der Füllstoffzusammensetzung und/oder der Kompositmasse und/oder des Gießwerkstücks ausgewählt ist aus einer Gruppe, die ein Rheologieadditiv, ein Pigment, einen Farbstoff, eine Farbpaste, ein Effektpigment, einen Emulgator, ein Biozid, einen Härter, einen Beschleuniger, einen Inhibitor, einen Füllstoff, ein Lösungsmittel, ein Polymer, ein Monomer, einen Polymerisationsstarter, ein Organosilan, einen Quervernetzer, einen Entschäumer, ein Benetzungsadditiv, ein Quarz- und/oder Quarzfolgeprodukt, eine Ca- und/oder Mg-haltige Komponente, eine Al-haltige Komponente, ein Konservierungsmittel, insbesondere ein Film- und/oder Topfkonservierungsmittel, einen anorganischen und/oder organischen Füllstoff umfasst, der sowohl mineralisch, amorph, oder biogen vorlegen kann. Es werden Füllstoffe bevorzugt, die aus der Gruppe der Quarze, Cristobalite, Feldspäte, Calcite, Dolomite, Wollastonite stammen und alleine vorliegen können oder in Mischung in jedem Verhältnis..

In einer bevorzugten Ausführungsform kann wenigstens eine Füllstoffzusammensetzung und/oder eine Kompositmasse und/oder ein Gießwerkstück und bevorzugt kann jede Füllstoffzusammensetzung und/oder Kompositmasse und/oder ein Gießwerkstück verknüpft sein mit einer Identifikationsgröße (einer sogenannten "Füllstoffzusammenesetzungs-ID" oder "Kompositmassen-ID" oder "Gießwerkstück-ID"), welche charakteristisch ist für die jeweilige Füllstoffzusammensetzung und/oder die jeweilige Kompositmasse und/oder das jeweilige Gießwerkstück. Die Identifikationsgröße (Allgemein als "ID" bezeichnet) kann auch charakteristisch sein für
- eine Charge der Füllstoffzusammensetzung und/oder der Kompositmasse und/oder des Gießwerkstücks und/oder
- einen Erzeuger der Füllstoffzusammensetzung und/oder der Kompositmasse und/oder des Gießwerkstücks und/oder
- einer Lagermodalität der Füllstoffzusammensetzung und/oder der Kompositmasse und/oder des Gießwerkstücks und/oder
- eine Transportmodalität der Füllstoffzusammensetzung und/oder der Kompositmasse und/oder des Gießwerkstücks (etwa ein Transportmittel und/oder ein Transportbehältnis und/oder eine Vorfüllung des Transportbehältnisses und/oder dergleichen).

Dadurch kann (bei identischer Rezeptur und/oder (Soll-) Zusammensetzung einer Füllstoffzusammensetzung und/oder einer Kompositmasse und/oder eines Gießwerkstücks) eine eventuell durch diese Faktoren hervorgerufene Abweichung einer Eigenschaft von einer Soll-Eigenschaft anhand der ID rückverfolgt werden und so auch bei der computer-implementierte Ermittlung des wenigstens einen Werts des ersten Zusammensetzungsdatensatzes berücksichtigt werden.

Bevorzugt wird der Zusammensetzungsdatensatz wenigstens einer Füllstoffzusammensetzung und/oder einer Kompositmasse und/oder eines Gießwerkstücks in Abhängigkeit der der jeweiligen Kompositmasse und/oder dem jeweiligen Gießwerkstück zugeordneten Identifikationsgröße (insbesondere der "Kompositmassen-ID" oder "Gießwerkstück-ID") erfasst, etwa durch Abrufen von einer Datenbank (welche beispielsweise mittels einer externen Speichereinrichtung und/oder einem externen Server bereitgestellt sein kann), in welcher bevorzugt eine Vielzahl von Paaren aus derartigen IDs und dieser ID jeweils zugeordnetem Zusammensetzungsdatensatz oder hierfür charakteristische Daten abgelegt ist.

Bevorzugt ist die Identifikationsgröße (ID, insbesondere die "Kompositmassen-ID" oder "Gießwerkstück-ID") einzigartig und/oder eindeutig und besonders bevorzugt eineindeutig für die jeweilige Füllstoffzusammensetzung und/oder der jeweiligen Kompositmasse und/oder das jeweilige Gießwerkstück. Denkbar wäre in diesem Zusammenhang beispielsweise, dass ein erster Zusammensetzungsdatensatz einer Füllstoffzusammensetzung und/oder einer Kompositmasse oder einem Gießwerkstück, welche/welches unter bestimmten Bedingungen für eine vorgegebene Zeit gelagert wurde eine erste Identifikationsgröße zugewiesen bekommt, wohingegen einer ursprünglich identischen Füllstoffzusammensetzung und/oder indentischen Kompositmasse oder identischem Gießwerkstück, welche/s unter anderen Bedingungen und/oder über eine andere Zeit gelagert wurde, eine andere Identifikationsgröße zugewiesen wird.

Dabei ist zu beachten, dass sich die Zusammensetzungsdatensätze zweier ursprünglich identischer Füllstoffzusammensetzungen oder Kompositmassen oder Gießwerkstücke unterscheiden können, da aufgrund der unterschiedlichen Lagerung und/oder Transportmodalität eine Analysegröße oder mehrere der Analysegrößen verändert sein könnten.

Analog unterscheiden sich die Zusammensetzungsdatensätze einer Füllstoffzusammensetzung und einer daraus erzeugten Kompositmasse und eines daraus hergestellten Gießwerkstücks, wobei es jedoch möglich und in einigen Ausführungsformen vorteilhaft ist, wenn ein Verweis zwischen diesen Zusammensetzungsdatensätzen besteht. Ein solcher Verweis enthält bevorzugt zusätzlich Informationen über das Verfahren zur Umsetzung dieser Kompositmasse zu diesem daraus hergestellten Gießwerkstück.

Das vorgeschlagene Verfahren, insbesondere die Verwendung eines reduzierten Datensatzes (durch Verwendung der jeweiligen Zusammensetzungsdatensätze zu den Füllstoffzusammensetzungen und/oder Kompositmassen und/oder Gießwerkstücken), bietet den Vorteil, dass es keine bzw. stark verringerte unbekannte Bereiche aufweist.

Diese Daten (der Zusammensetzungsdatensätze) werden vorzugsweise standardmäßig bei der Herstellung der jeweiligen Füllstoffzusammensetzung und/oder der jeweiligen Kompositmassen und/oder Gießwerkstücke bereitgestellt. Die Menge an zu übermittelnden Daten ist gegenüber den Einzelkomponenten erheblich kleiner. Durch die bevorzugte Verwendung von funktional aggregierten Gruppen wird ein robusteres Ergebnis erzeugt.

Bei einem weiter bevorzugten Verfahren erfolgt die Ermittlung der wenigstens einen Eigenschaft bzw. einer dazu korrelierenden Analysegröße auf Grundlage von zu einer Vielzahl verschiedener (Trainings-) Füllstoffzusammensetzungen und/oder (Trainings-) Kompositmassen und/oder (Trainings-) Gießwerkstücken jeweils erfassten Zusammensetzungsdatensätzen, welche insbesondere Informationen zu den darin jeweils enthaltenen Komponenten sowie deren jeweilige Anteilsgröße in der jeweiligen Füllstoffzusammensetzung und/oder der jeweiligen Kompositmasse und/oder dem jeweiligen Gießwerkstück umfassen. Ein Zusammensetzungsdatensatz ist damit vorzugsweise geeignet, eine Füllstoffzusammensetzung und/oder eine Kompositmasse und/oder ein Gießwerkstück hinsichtlich mindestens einer Eigenschaft und mindestens zwei Komponenten sowie deren jeweilige Anteilsgröße zu charakterisieren.

Der Ausdruck "Trainings-Füllstoffzusammensetzung" und/oder "Trainings-Kompositmasse" und/oder "Trainings-Gießwerkstück" ist insbesondere so zu verstehen, dass sich diese Trainings-Füllstoffzusammensetzung bzw. diese Trainings-Kompositmasse bzw. dieses Trainings-Gießwerkstück von der Füllstoffzusammensetzung und/oder der Kompositmasse und/oder dem Gießwerkstück unterscheidet, von der die wenigstens eine (physiko-chemische) Eigenschaft ermittelt werden soll. Vorzugsweise eine Vielzahl derartiger "Trainings-Füllstoffzusammensetzungen" und/oder "Trainings-Kompositmassen" und/oder "Trainings-Gießwerkstücke" werden insbesondere im zeitlichen Vorlauf zur Ermittlung der (Soll-) Eigenschaft der ersten Füllstoffzusammensetzung und/oder der ersten Kompositmasse und/oder dem ersten Gießwerkstück ausgewertet (und beispielsweise analysiert und/oder Rezepturdaten herangezogen) und auf Grundlage deren Auswertung kann auf eine Eigenschaft der Füllstoffzusammensetzung und/oder der Kompositmasse und/oder des Gießwerkstücks geschlossen werden. Insbesondere handelt es sich bei den Trainings-Füllstoffzusammensetzungen und/oder den Trainings-Kompositmassen und/oder Trainings-Gießwerkstücken insofern um bekannte Füllstoffzusammensetzungen und/oder Kompositmassen und/oder Gießwerkstücke, von denen Messdaten bzw. Messgröße(n) oder hiervon abgeleitete Eigenschaftsgröße(n) vorliegen, welche für die wenigstens eine Analysegröße charakteristisch sind.

Das vorgeschlagene Verfahren bietet im Vergleich zu dem aus dem eingangs beschriebenen Stand der Technik bekannten Verfahren den Vorteil verringerter Kosten bei der Bereitstellung der Daten. Weiter vorteilhaft kann ein Zurückgreifen bei der Datenbasis auf einen etablierten und angewandten Standard erfolgen. Durch das vorgeschlagene Verfahren kann ein robustes Ergebnis erreicht werden und Parameter (insbesondere für die (Soll-) Eigenschaft) ermittelt werden, die sonst nicht rechnerisch vorhersagebar wären bzw. mittels einer auf Einzelkomponenten basierenden Ermittlung fehleranfälliger und/oder mit größeren Abweichen verhaftet wären.

Bevorzugt wird auf Grundlage der Vielzahl von der zu den Trainings-Füllstoffzusammensetzungen und/oder den Trainings-Kompositmassen und/oder Trainings-Gießwerkstücken ermittelten bzw. erfassten Zusammensetzungsdatensätzen sowie auf Grundlage der diesen zugeordneten Analysegrößen, welche charakteristisch sind für wenigstens eine Eigenschaft dieser (Trainings-) Füllstoffzusammensetzung und/oder (Trainings-) Kompositmassen und/oder (Trainings-) Gießwerkstücke, ein insbesondere auf einem (künstlichen) neuronalen Netz basierenden und/oder eines Kompositmassenrezeptierungsmodells maschinellen Lernens zur Ermittlung wenigstens einer Eigenschaft einer Füllstoffzusammensetzung und/oder einer Kompositmasse und/oder einem Gießwerkstück trainiert. Dieses Kompositmassenrezeptierungsmodells wird nachfolgend näher beschrieben. Bevorzugt wird dabei ein nachfolgend näher beschriebenes Kompositmassenrezeptierungsmodell zur Ermittlung wenigstens einer (physiko-chemischen) Eigenschaft einer (ersten) Füllstoffzusammensetzung und/oder einer (ersten) Kompositmasse und/oder einem (ersten) Gießwerkstück gemäß einer bevorzugten Ausführungsform zur Ermittlung der wenigstens einen Eigenschaftsgröße verwendet.

Bevorzugt basiert das Kompositmassenrezeptierungsmodell maschinellen Lernens auf einem (künstlichen) neuronalen Netzwerk. Bevorzugt ist das neuronale Netzwerk als tiefes neuronales Netzwerk (Deep Neural Network, DNN), bei dem die parametrierbare Verarbeitungskette eine Mehrzahl von Verarbeitungsschichten aufweist, und/oder ein sogenanntes Convolutional Neural Network (CNN) (dt. Faltungsnetzwerk) und/oder ein rekurrentes Neuronales Netzwerk (RNN, engl. Recurrent Neural Network) ausgebildet. Besonders bevorzugt basiert das Kompositmassenrezetierungsmodell auf einem Ensemble-Lernverfahren auf Basis eines Random Forest Modells (RF). Random Forest arbeitet mit Entscheidungsbaumstrukturen, die bestimmte Regeln aufstellen und gesondert trainiert werden können.

Bevorzugt bezieht sich die vorliegende Erfindung auf ein Al-ML-Verfahren zur Bestimmung von mindestens einer Eigenschaft einer Füllstoffzusammensetzung und/oder einer Kompositmasse und/oder einem Gießwerkstück.

Bei einem weiter bevorzugten Verfahren erfolgt die Ermittlung der wenigstens einen (physiko-chemischen) Eigenschaft einer (ersten) Füllstoffzusammensetzung und/oder einer (ersten) Kompositmasse und/oder einem (ersten) Gießwerkstück (beispielsweise mittels einer Kompositmassenrezept-Ermittlungsvorrichtung) in Abhängigkeit eines, insbesondere trainierbaren, Kompositmassenrezeptierungsmodells maschinellen Lernens, welches einen Satz insbesondere trainierbarer, Parameter umfasst, welche auf Werte eingestellt sind, die als Ergebnis eines Trainingsprozesses gelernt wurden, wobei der Trainingsprozess auf Grundlage eines Satzes von Trainingsdaten erfolgt ist.

Bevorzugt umfasst das Verfahren weiterhin die Erzeugung eines ersten Verfahrensdatensatzes, der Daten betreffend ein Verfahren zur Umsetzung einer ersten Füllstoffzusammensetzung zu einer ersten Kompositmasse und weiter zu einem ersten Gießwerkstück umfasst. Die Rezeptur der ersten Füllstoffzusammensetzung bzw. der daraus resultierenden Kompositmasse kann nach einem wie oben beschriebenen Verfahren ermittelt worden sein.

Bevorzugt handelt es sich also bei der Kompositmasse um die erste Kompositmasse, die durch den ersten Zusammensetzungsdatensatz aus einer Füllstoffzusammensetzung charakterisierbar ist.

Es ist bekannt, dass verschiedene Verfahren der Umsetzung einer Füllstoffzusammensetzung zu einer Kompositmasse und weiter zu einem Gießwerkstück zu verschiedenen Eigenschaften des Gießwerkstücks führen können. Dementsprechend können einer Füllstoffzusammensetzung und einer ersten Kompositmasse mehrere Verfahrensdatensätze zugeordnet sein. Die Umsetzung einer solchen ersten Füllstoffzusammensetzung zu einer Kompositmasse gemäß eines durch den jeweiligen Verfahrensdatensatz charakterisierten Verfahrens kann dann jeweils zu einem anderen Gießwerkstück führen, wobei sie die so erhaltenen Gießwerkstück in ihren Eigenschaften unterscheiden können.

Das Verfahren zur Erzeugung des ersten Verfahrensdatensatzes umfasst die Schritte:
- Erfassung eines zweiten Verfahrensdatensatzes, der Daten betreffend ein Verfahren zur Umsetzung einer zweiten Füllstoffzusammensetzung zu einer zweiten Kompositmasse zu einem zweiten Gießwerkstück umfasst, wobei der zweite Verfahrensdatensatz mindestens einen Wert enthält, welcher für einen Prozessparameter charakteristisch ist, der einen Effekt auf die durch die erste Analysegröße charakterisierte erste Eigenschaft einer Kompositmasse und/oder einem Gießwerkstück hat,
- Erfassung mindestens eines weiteren Verfahrensdatensatzes, der jeweils Daten betreffend ein Verfahren zur Umsetzung einer weiteren Füllstoffzusammensetzung zu einer weiteren Kompositmasse zu einem weiteren Gießwerkstück umfasst, wobei der weitere Verfahrensdatensatz mindestens einen Wert enthält, welcher für einen Prozessparameter charakteristisch ist, der einen Effekt auf die durch die erste Analysegröße charakterisierte erste Eigenschaft einer Füllstoffzusammensetzung und/oder einer Kompositmasse und/oder einem Gießwerkstück hat,
- Computer-implementierte Ermittlung von wenigstens einem Verfahrensdatenwert des ersten Verfahrensdatensatzes, auf Grundlage des zweiten Verfahrensdatensatzes und mindestens eines weiteren Verfahrensdatensatzes, wobei dieser Verfahrensdatenwert für einen Prozessparameter charakteristisch ist, dessen Einstellung von einem anderen Wert hin zu dem durch den Verfahrensdatenwert charakterisierten Wert zumindest eine Annäherung der ersten Analysegröße hin zu einer Soll-Analysegröße ermöglicht, welche eine Soll-Eigenschaft eines Gießwerkstücks charakterisiert.

Durch diese Verfahrensvariante ist es vorzugsweise möglich, basierend auf mehreren Verfahrensdatensätzen mit Unterstützung der Computer-implementierten Ermittlung von wenigstens einem Verfahrensdatenwert eine Prozessoptimierung dahingehend zu ermitteln, dass ein mit dem veränderten Prozess hergestelltes Gießwerkstück eine Eigenschaft aufweist, deren Messung eine Analysegröße als Ergebnis liefert, die näher an einer Soll-Analysegröße liegt als bei einer Messung dieser Eigenschaft eines nach einem anderen Prozesses hergestellten Gießwerkstücks. Somit ist eine Optimierung der Umsetzung einer ersten Füllstoffzusammensetzung zu einer ersten Kompositmasse zu einem ersten Gießwerkstück zumindest hinsichtlich der betrachteten, durch die erste Analysegröße charakterisierte erste Eigenschaft möglich. Die kann ohne, oder zumindest mit deutlich reduzierten und/oder weniger langwierigen Experimenten und Laboruntersuchungen erfolgen. Es können daher Zeit und Ressourcen eingespart werden.

Bevorzugt ist eine Verfahrensvariante, bei der während der computer-implementierten Ermittlung des wenigstens einen Verfahrensdatenwert des ersten Verfahrensdatensatzes der ermittelte wenigstens eine Verfahrensdatenwert charakteristisch ist für einen Prozessparameter, der ausgewählt ist aus einer Gruppe, die eine Zeit, eine Temperatur, einen Druck, ein Werkzeug, eine Vorbehandlung eines Werkzeugs, eine Art einer Applikation eines Reagenzes und eine Reihenfolge der Zugabe von Reagenzien umfasst. Diese Prozessparameter haben sich als besonders geeignet gezeigt, einen (positiven) Effekt auf eine Vielzahl von Eigenschaften eines Gießwerkstücks zu haben. Daher ist es durch die Anpassung eines einen solchen Prozessparameter charakterisierenden Verfahrensdatenwert besonders gut möglich, eine Analysegröße hin zu einer Soll-Analysegröße zu verändern.

Bevorzugt umfasst der zweite Verfahrensdatensatz und/oder mindestens einer der weiteren Verfahrensdatensätze jeweils mindestens 3 Verfahrensdatenwerte, die für einen Prozessparameter zur Umsetzung einer zweiten oder weiteren Kompositmasse zu einem zweiten oder weiteren Gießwerkstück charakteristisch sind. Bevorzugt umfasst der zweite Verfahrensdatensatz und/oder mindestens einer der weiteren Verfahrensdatensätze unabhängig von anderen Verfahrensdatensätzen mindestens 5, weiter bevorzugt mindestens 7, meist bevorzugt mindestens 10 Verfahrensdatenwerte. Dadurch wird ermöglicht, dass mehrere, sich eventuell gegenseitig beeinflussende Prozessparametergemeinsam betrachtet und/oder gewichtet werden können. Es wird dadurch möglich, mehrere Verfahrensdatenwerte gemeinsam und bevorzugt unter gegenseitiger Berücksichtigung zu wie oben beschrien zu optimieren, so dass eine nochmals verbesserte Anpassung der dadurch charakterisierten Prozessparameter möglich wird und damit auch eine verbesserte Annäherung der Analysegröße hin zu einer Soll-Analysegröße.

Die oben beschriebenen Verfahrensdatenwerte sind bevorzugt während einer Umsetzung einer zweiten oder weiteren Kompositmasse zu einem zweiten oder weiteren Gießwerkstück bestimmt worden. Oftmals werden bereits einige Prozessparameter während einer Umsetzung einer Kompositmasse zu einem Gießwerkstück bestimmt. Diese Werte werden oftmals zu Dokumentationszwecken und/oder zur Qualitätssicherung gespeichert. Durch eine Bestimmung weiterer Verfahrensdatenwerte und/oder den Zugriff auf bereits bestehende Datenbanken ist es möglich, Verfahrensdatensätze zur Nutzung in dieser Verfahrensvariante bereitzustellen. Daneben oder ergänzend dazu wäre es jedoch auch möglich einzelne oder mehrere Verfahrensdatensätze und/oder einzelne Verfahrensdatenwerte eines oder mehrerer Verfahrensdatensätze nachträglich zu ergänzen, beispielsweise durch Extrapolation, Abschätzung und/oder die Nutzung eines dafür geeigneten Systems künstlicher Intelligenz.

Für den Schritt der computer-implementierten Ermittlung des wenigstens einen Verfahrensdatenwert des ersten Verfahrensdatensatzes wird vorzugsweise ein Verfahrensdatenwert-Ermittlungsmodell genutzt. Bevorzugt basiert ein solches Verfahrensdatenwert-Ermittlungsmodell maschinellen Lernens auf einem (künstlichen) neuronalen Netzwerk. Bevorzugt ist das neuronale Netzwerk als tiefes neuronales Netzwerk (Deep Neural Network, DNN), bei dem die parametrierbare Verarbeitungskette eine Mehrzahl von Verarbeitungsschichten aufweist, und/oder ein sogenanntes Convolutional Neural Network (CNN) (dt. Faltungsnetzwerk) und/oder ein rekurrentes Neuronales Netzwerk (RNN, engl. Recurrent Neural Network) ausgebildet. Besonders bevorzugt basiert das Verfahrensdatenwert-Ermittlungsmodell maschinellen Lernens auf Basis eines Random Forest Modells (RF). Random Forest Modelle arbeiten mit Entscheidungsbaumstrukturen, die bestimmte Regeln aufstellen und gesondert trainiert werden können. Diese Art von Modellen kommen sehr gut mit kleinen Datensätzen aus und die Entscheidungsfindung ist sehr gut nachvollziehbar.

Das Verfahrensdatenwert-Ermittlungsmodell maschinellen Lernens wird/wurde bevorzugt unter Verwendung vorgegebener Trainingsdaten trainiert, wobei durch das Training die parametrierbare Verarbeitungskette parametriert wird. Bevorzugt schließen die Trainingsdaten die oben erwähnten Verfahrensdatenwerte ein, die während vorheriger Umsetzungen von (bevorzugt Füllstoffzusammensetzungen zu) Kompositmassen (und weiter) zu Gießwerkstücken bestimmt wurden.

Es ist auch möglich, dass das Verfahrensdatenwert-Ermittlungsmodell Teil des unten näher beschriebenen Kompositmassenrezeptierungmodell ist und dieses somit auch Informationen über mögliche Verfahren zur Umsetzung von (bevorzugt Füllstoffzusammensetzungen zu) Kompositmassen (und weiter) zu Gießwerkstücken umfasst.

Außerdem ist es möglich und in einigen Ausführungsformen bevorzugt, dass das Verfahrensdatenwert-Ermittlungsmodell und das Kompositmassenrezeptierungmodell sich in einem oder mehreren Aspekten ähneln beispielsweise analog aufgebaut, trainiert, gespeichert, bereitgestellt etc. sind. Dementsprechend sollen alle unten zu dem Kompositmassenrezeptierungmodell beschriebenen Ausführungsformen und verfahren individuell auch als für das Verfahrensdatenwert-Ermittlungsmodell als offenbart gelten.

Analog gilt dies auch für eine hier nicht näher beschriebene Verfahrensdatenwertermittlungsvorrichtung, die bevorzugt analog der unten beschriebenen Kompositmassenrezept-Ermittlungsvorrichtung aufgebaut und eingerichtet sein kann. Dabei müssen bei der Verfahrensdatenwertermittlungsvorrichtung nicht alle unten für die Kompositmassenrezept-Ermittlungsvorrichtung beschriebenen Aspekte gemeinsam und/oder analog zu denen der Kompositmassenrezept-Ermittlungsvorrichtung realisiert sein. Es ist vielmehr möglich, dass bei der Verfahrensdatenwertermittlungsvorrichtung unabhängig von der Kompositmassenrezept-Ermittlungsvorrichtung lediglich einige der unten für die Kompositmassenrezept-Ermittlungsvorrichtung beschriebenen Aspekte realisiert sind, andere jedoch nicht.

Bevorzugt wird/wurde das Kompositmassenrezeptierungsmodell maschinellen Lernens unter Verwendung vorgegebener Trainingsdaten trainiert, wobei durch das Training die parametrierbare Verarbeitungskette parametriert wird.

Bevorzugt umfassen die Trainingsdaten wenigstens einen zweiten Zusammensetzungsdatensatz zu einer zweiten Füllstoffzusammensetzung und/oder einer zweiten Kompositmasse und/oder einem zweiten Gießwerkstück, wobei der zweite Zusammensetzungsdatensatz wenigstens eine Analysegröße und eine Anteilsgröße einer eine zweite Analysegröße und eine Anteilsgröße einer zweiten Komponente und bevorzugt einer Vielzahl von Anteilsgrößen von weiteren Komponenten umfasst, sowie mindestens einen weiteren Zusammensetzungsdatensatzes zu mindestens einer weiteren Füllstoffzusammensetzung und/oder einer weiteren Kompositmasse und/oder einem weiteren Gießwerkstück, wobei der mindestens eine weitere Zusammensetzungsdatensatz wenigstens eine weitere Analysegröße und eine weitere Anteilsgröße einer weiteren Komponente und bevorzugt eine Vielzahl von weiteren Anteilsgrößen von weiteren Komponenten umfasst. Bevorzugt ist die zweite und/oder weitere Analysegröße jeweils charakteristisch ist für wenigstens eine Eigenschaft der/des durch den jeweiligen Zusammensetzungsdatensatz charakterisierten Füllstoffzusammensetzung und/oder Kompositmasse und/oder Gießwerkstücks.

Bevorzugt werden dem Kompositmassenrezeptierungsmodell bzw. dem (künstlichen) neuronalen Netzwerk die (zu verarbeitenden) Daten, insbesondere der oben genannten erste Analysedatensatz und der mindestens eine weitere Zusammensetzungsdatensatz (oder hiervon abgeleitete Daten) als Eingangsgrößen zugeführt. Bevorzugt bildet das Kompositmassenrezeptierungsmodell bzw. das künstliche neuronale Netzwerk bzw. das Random Forest Modell die Eingangsgrößen in Abhängigkeit einer parametrierbaren Verarbeitungskette auf Ausgangsgrößen ab.

Bevorzugt ist als Ausgangsgröße wenigstens eine Größe gewählt, die für eine Eigenschaft der Füllstoffzusammensetzung und/oder der Kompositmasse und/oder des Gießwerkstücks (beispielsweise ein Dichtewert, ein Viskositätswert) charakteristisch ist.

Es ist jedoch auch denkbar, einen Soll-Wert für eine (physiko-chemischen) Eigenschaft der Füllstoffzusammensetzung und/oder der Kompositmasse und/oder des Gießwerkstücks vorzugeben und das Verfahren zu nutzen, um Deskriptoren für eine Füllstoffzusammensetzung und/oder eine Kompositmasse und/oder ein Gießwerkstück zu ermitteln, welche (gemäß dieser Ermittlung wahrscheinlich) den vorgegebenen Soll-Wert der Eigenschaft aufweist. Bei dieser Variante des Verfahrens wird somit ein Soll-Wert der (physiko-chemischen) Eigenschaft vorgegeben und die Komponenten der Füllstoffzusammensetzung und/oder der Kompositmasse und/oder des Gießwerkstücks sowie die diesen Komponenten zugehörigen Anteilsgrößen ermittelt. Diese Verfahrensvariante ist besonders dann vorteilhaft, wenn nach Formulierungen für eine Füllstoffzusammensetzung und/oder einer Kompositmasse und/oder ein Gießwerkstück gesucht wird, die eine bestimmte Soll-Eigenschaft aufweisen soll.

Bei einem bevorzugten Verfahren werden in dem Trainingsprozess des Kompositmassenrezeptierungsmodells Trainingsdaten verwendet, welche zumindest eine Analysegröße und für Komponenten einer Füllstoffzusammensetzung und/oder einer Kompositmasse und/oder einem Gießwerkstück charakteristische Größen sowie jeweils diesen Komponenten zugeordnete Anteilsgrößen umfassen. Diese Daten können im Rahmen einer Analyse ermittelt sein (bevorzugt für die Analysegröße) und/oder im Rahmen der Herstellung der Kompositmassen und/oder Gießwerkstücke, auf denen die Trainingsdaten beruhen (beispielsweise aus der Rezeptur oder Überwachungseinrichtungen der genutzten Gerätschaften (bevorzugt für die Anteilsgrößen und den zugehörigen Komponenten der Füllstoffzusammensetzung und/oder der Kompositmasse und/oder dem Gießwerkstück) umfassen. Denkbar ist auch, dass Trainingsdaten auf Grundlage von Füllstoffzusammensetzung und/oder von Kompositmassen und/oder Gießwerkstücken verschiedener Chargen (optional verschiedener Anbieter und/oder Komponenten aus verschiedenen Quellen) verwendet werden. Dies bietet den Vorteil, dass in kurzer Zeit eine hohe Zahl an Trainingsdaten erzeugt werden kann.

Bevorzugt werden die zur Verwendung als Trainingsdaten vorgesehenen ermittelten Analysegrößen mit weiteren Merkmalen wie beispielsweise einem Analysemerkmal (welches beispielsweise eine oder mehr Größen umfasst, die für die Art, Methode, Dienstleiser der/die für die Ermittlung dieser Analysegröße charakteristisch ist), einem Herstellermerkmal, einem Lagermerkmal (welches beispielsweise eine oder mehr Größen umfasst, die für die Art, Dauer, Temperatur, Lagerbehältnis oder eine andere lagerbedingte Eigenschaft charakteristisch ist) und/oder einem Transportmerkmal (welches beispielsweise eine oder mehr Größen umfasst, die für die Art, Dauer, Strecke, Temperatur, Transportbehältnis oder eine andere transportbedingte Eigenschaft charakteristisch ist) versehen.

Bevorzugt werden die erfassten Analysedatensätze, optional zusammen mit den jeweils ihnen zugeordneten weiteren Merkmalen (beispielsweise Analysemerkmal, Herstellermerkmal, Lagermerkmal und/oder Transportmerkmal als Trainingsdatensatz (insbesondere auf einer und/oder der nicht-flüchtigen Speichereinrichtung) abgelegt und/oder verwendet. Bevorzugt wird auf diese Weise eine Vielzahl von Trainings-Datensätzen bereitgestellt.

Durch die Variation der Anteilsgrößen einer oder mehrerer Komponente/n und die Korrelation zu der oder den Analysegrößen ist es theoretisch möglich, eine Korrelation zwischen den Anteilsgrößen einer oder mehrerer Komponente/n und den Analysegrößen beziehungsweise einer Eigenschaft einer Füllstoffzusammensetzung und/oder einer Kompositmasse und/oder einem Gießwerkstück zu ermitteln.

Bevorzugt wird ein derart trainiertes neuronales Netzwerk (als Kompositmassenrezeptierungsmodell) eingesetzt. Besonders bevorzugt wird ein Random Forest Modell eingesetzt. Bevorzugt erfolgt das Training mittels überwachtem Lernen. Es wäre jedoch auch möglich, das Kompositmassenrezeptierungsmodell bzw. das Kl-basierte Netzwerk / Modell mittels unbeaufsichtigtem Lernen, bestärkendem Lernen oder stochastischem Lernen zu trainieren.

Bevorzugt werden dem Kompositmassenrezeptierungsmodell die (zu verarbeitenden) Daten, insbesondere der erste und der mindestens eine weitere Zusammensetzungsdatensatz, (oder hierfür charakteristische oder hiervon abgeleitete Daten) als Eingangsgrößen zugeführt. Bevorzugt bildet das Kompositmassenrezeptierungsmodell die Eingangsgrößen in Abhängigkeit einer parametrierbaren Verarbeitungskette auf Ausgangsgrößen ab.

Bevorzugt ist als Ausgangsgröße wenigstens eine Eigenschaft einer Füllstoffzusammensetzung und/oder einer Kompositmasse und/oder eines Gießwerkstücks und/oder eine für eine Füllstoffzusammensetzung und/oder eine Kompositmasse und/oder ein Gießwerkstück charakteristische Größe gewählt.

Bevorzugt umfasst die Ausgangsgröße auch Daten, die charakteristisch sind für eine erste Komponente und wenigstens eine weitere, von der ersten Komponente verschiedenen zweite Komponente und weiter bevorzugt auch deren jeweilige Anteilsgrößen in einer Füllstoffzusammensetzung und/oder einer Kompositmasse und/oder einem Gießwerkstück und/oder für diesen Anteil charakteristische Größen.

Bevorzugt wird/wurde das Kompositmassenrezeptierungsmodell maschinellen Lernens unter Verwendung vorgegebener Trainingsdaten trainiert, wobei durch das Training die parametrierbare Verarbeitungskette parametriert wird.

Bei einem bevorzugten Verfahren werden in dem Trainingsprozess des Kompositmassenrezeptierungsmodell Trainingsdaten verwendet, welche historisch erfasste Daten umfassen, insbesondere für Rezepturen von Füllstoffzusammensetzung und/oder von Kompositmassen und/oder Gießwerkstücken und mindestens eine (physiko-chemische) Eigenschaft einer Füllstoffzusammensetzung und/oder einer Kompositmasse und/oder eines Gießwerkstücks. Dies bietet den Vorteil, dass in kurzer Zeit eine hohe Zahl an Trainingsdaten erzeugt werden können.

Bevorzugt umfassen die Trainingsdaten als Klassifizierungsmerkmal die Information, ob eine Analysegröße einen Sollwert einnimmt (beziehungsweise innerhalb eines Sollwertbereichs liegt) und/oder ob eine Analysegröße einen Sollwert nicht einnimmt (beziehungsweise außerhalb eines Sollwertbereichs liegt).

Bei einem weiter bevorzugten Verfahren wird die ermittelte Eigenschaftsgröße zur Übermittlung an eine Ausgabeeinrichtung, beispielsweise einen Bildschirm und/oder ein (insbesondere mobiles) Endgerät eines Nutzers gesendet. Ergänzend oder alternativ dazu könnte die ermittelte Eigenschaftsgröße zum Ablegen auf einer (bzw. der obig beschriebenen) externen und/oder Cloud-basierten Speichereinrichtung (insbesondere mit einer (erzeugten), der Füllstoffzusammensetzung und/oder der Kompositmasse und/oder dem Gießwerkstück zugeordneten Füllstoffzusammensetzungs-ID, Kompositmassen-ID oder Gießwerkstück-ID) bereitgestellt werden und bevorzugt zu einem späteren Zeitpunkt an einen Nutzer mittels einer Ausgabeeinrichtung ausgegeben werden.

Bevorzugt liegt für jede hergestellte, bevorzugt für jede Füllstoffzusammensetzung und/oder jede Kompositmasse und/oder jedes Gießwerkstück eine einzigartige Identifikationsgröße (Füllstoffzusammensetzungs-ID, Kompositmassen-ID oder Gießwerkstück-ID) vor, wie obig bereits beschrieben. Die bei der Herstellung der Füllstoffzusammensetzung und/oder der Kompositmassen und/oder Gießwerkstücken (insbesondere typischerweise ohnehin standardmäßig) ermittelten bzw. erzeugten Analysedatensätze sind bevorzugt zusammen mit der Identifikationsgröße in der (obig beschriebenen) Datenbank abgelegt, bevorzugt auf einem (in Bezug auf die Kompositmassenrezept-Ermittlungsvorrichtung) externen Server.

Weiterhin ist die Erfindung gerichtet auf ein Computer-implementiertes Verfahren zur Erzeugung eines trainierten Kompositmassenrezeptierungsmodells maschinellen Lernens zur Ermittlung wenigstens einer ersten Eigenschaft einer ersten Füllstoffzusammensetzung und/oder einer ersten Kompositmasse und/oder eines ersten daraus herstellbaren Gießwerkstück auf Basis einer Verwendung von Zusammensetzungsdatensätzen, welche jeweils für eine Füllstoffzusammensetzung und/oder eine Kompositmasse und/oder ein Gießwerkstück charakteristisch sind, wobei der Zusammensetzungsdatensatz zu der jeweiligen Kompositmasse und/oder dem jeweiligen Gießwerkstück jeweils wenigstens eine Analysegröße und eine Anteilsgröße einer Komponente umfasst, wobei die Analysegröße jeweils charakteristisch ist für wenigstens eine Eigenschaft der/des durch den jeweiligen Zusammensetzungsdatensatz charakterisierten Füllstoffzusammensetzung und/oder Kompositmasse und/oder Gießwerkstücks, umfassend:
- Bereitstellen eines trainierbaren Kompositmassenrezeptierungsmodells maschinellen Lernens, welches einen Satz trainierbarer Parameter umfasst und welches auf Grundlage von zu mindestens einem zu einer Füllstoffzusammensetzung und/oder einer Kompositmasse und/oder einem Gießwerkstück erfassten Zusammensetzungsdatensatz oder hiervon abgeleiteter Daten als Eingangsgrößen wenigstens eine für eine Eigenschaft der ersten Füllstoffzusammensetzung und/oder der ersten Kompositmasse und/oder des ersten Gießwerkstück charakteristische Analysegröße als Ausgangsgröße erzeugt;
- Erzeugen eines Trainingsdatensatzes umfassend eine Vielzahl erfasster und/oder ermittelter Zusammensetzungsdatensätze zu Trainings-Füllstoffzusammensetzungen und/oder zu Trainings-Kompositmassen und/oder Trainings-Gießwerkstücken, welche zum Erzeugen der Trainingsdatensätzen verwendet werden, sowie Messdaten oder eine hiervon abgeleitete Analysegrößen, welche charakteristisch sind bzw. ist für wenigstens eine Eigenschaft der jeweiligen Trainings-Füllstoffzusammensetzungen und/oder Trainings-Kompositmasse und/oder Trainings-Gießwerkstück;

Trainieren des Kompositmassenrezeptierungsmodells auf Grundlage des Trainingsdatensatzes.

Bevorzugt wird das so erzeugte Kompositmassenrezeptierungsmodell zur Ermittlung wenigstens einer für wenigstens eine Eigenschaft einer ersten Füllstoffzusammensetzung und/oder einer ersten Kompositmasse und/oder einem ersten Gießwerkstück charakteristischen Analysegröße verwendet. Dies kann bevorzugt im Rahmen eine oben beschriebenen Verfahrens erfolgen und/oder durch eine wie im Folgenden beschriebene Kompositmassenrezept-Ermittlungsvorrichtung.

Die vorliegende Erfindung ist weiterhin gerichtet auf eine, bevorzugt Prozessor-basierte, Kompositmassenrezept-Ermittlungsvorrichtung zur Ermittlung wenigstens einer (bevorzugt physiko-chemischen) Eigenschaft einer ersten Füllstoffzusammensetzung und/oder einer ersten Kompositmasse und/oder einem ersten Gießwerkstück.

Erfindungsgemäß ist die Kompositmassenrezept-Ermittlungsvorrichtung dazu geeignet und bestimmt, einen zweiten Zusammensetzungsdatensatz zu einer zweiten Füllstoffzusammensetzung und/oder einer zweiten Kompositmasse und/oder einem zweiten Gießwerkstück und mindestens einen weiteren Zusammensetzungsdatensatz zu mindestens einer weiteren Füllstoffzusammensetzung und/oder einer weiteren Kompositmasse und/oder einem weiteren Gießwerkstück zu erfassen, wobei der jeweilige Zusammensetzungsdatensatz wenigstens eine zweite oder weitere Analysegröße und wenigstens eine zweite oder weitere Anteilsgröße einer zweiten oder weiteren Komponente und bevorzugt einer Vielzahl von weiteren Anteilsgrößen von zweiten oder weiteren Komponenten umfasst, wobei die Analysegröße jeweils charakteristisch ist für wenigstens eine Eigenschaft einer zweiten oder weiteren Füllstoffzusammensetzung und/oder einer weiteren oder zweiten Kompositmasse und/oder einem zweiten oder weiteren Gießwerkstück (GWS₂₋ₙ), und wobei die Anteilsgröße jeweils charakteristisch ist für einen Mengenanteil der in der jeweiligen Füllstoffzusammensetzung und/oder der jeweiligen Kompositmasse und/oder dem jeweiligen Gießwerkstück befindlichen zweiten und/oder weiteren zweiten oder weiteren Komponente, wobei die Kompositmassenrezept-Ermittlungsvorrichtung dazu geeignet und bestimmt ist, mittels einer Prozessoreinrichtung wenigstens einen Wert eines Zusammensetzungsdatensatzes, welcher Daten für die Rezeptierung einer ersten Füllstoffzusammensetzung oder einer ersten Kompositmasse für die Herstellung eines ersten Gießwerkstück auf Grundlage des zweiten Zusammensetzungsdatensatz zu der zweiten Füllstoffzusammensetzung und/oder zu der zweiten Kompositmasse und/oder dem zweiten Gießwerkstück und dem mindestens einen weiteren Zusammensetzungsdatensatz zu der mindestens einen weiteren Kompositmasse (und/oder dem weiteren Gießwerkstück zu ermitteln.

Bevorzugt ist der Zusammensetzungsdatensatz jeweils entsprechend einer obig beschriebenen (bevorzugten) Ausführungsform ausgestaltet.

Es wird also auch im Rahmen der erfindungsgemäßen Kompositmassenrezept-Ermittlungsvorrichtung vorgeschlagen, dass durch die Verwendung der Zusammensetzungsdatensätze bereits weitegehend vorliegende (und gegebenenfalls durch Analysedaten ergänzte) Datensätze verwendet werden, insbesondere solche, welche standardmäßig bei der Herstellung von Füllstoffzusammensetzungen und/oder Kompositmassen und/oder Gießwerkstücken bereitgestellt bzw. (im laufenden Prozess) bestimmt werden. Die Menge an zu übermittelnden Daten ist gegenüber den Einzelkomponenten erheblich kleiner. Durch die obig im Rahmen des Verfahrens beschriebenen Verwendung von funktional aggregierten Gruppen wird vorteilhaft ein robusteres Ergebnis erzeugt.

Bevorzugt ist die Kompositmassenrezept-Ermittlungsvorrichtung dazu eingerichtet, geeignet und/oder bestimmt, das obig beschriebene Verfahren zur Erzeugung eines ersten Zusammensetzungsdatensatzes, welcher Daten für die Rezeptierung einer ersten Kompositmasse für die Herstellung eines ersten Gießwerkstück umfasst, sowie einen oder alle bereits obig im Zusammenhang mit dem Verfahren (oder einer bevorzugten Ausführungsform des Verfahrens) beschriebene Verfahrensschritte einzeln oder in Kombination miteinander auszuführen. Umgekehrt kann das Verfahren mit allen im Rahmen der Kompositmassenrezept-Ermittlungsvorrichtung beschriebenen Merkmalen einzeln oder in Kombination miteinander durchgeführt werden.

Weiterhin ist die Kompositmassenrezept-Ermittlungsvorrichtung bevorzugt dazu geeignet und bestimmt, ein gemäß des oben beschriebenen Verfahrens erzeugtes trainiertes Kompositmassenrezeptierungsmodell (bevorzugt in Bezug auf einen ersten Zusammensetzungsdatensatz, der eine erste Füllstoffzusammensetzung und/oder der ersten Kompositmasse und/oder ein erstes Gießwerkstück charakterisiert) zu verwenden. Bevorzugt wird hierzu dem Kompositmassenrezept-Ermittlungsvorrichtung ein zweiter Zusammensetzungsdatensatz und mindestens ein weiterer Zusammensetzungsdatensatz zugeführt. Hierzu ist die Kompositmassenrezept-Ermittlungsvorrichtung bevorzugt dazu geeignet und bestimmt, aus den dem Kompositmassenrezept-Ermittlungsvorrichtung zuzuführenden Analysedatensätzen einen Zusammensetzungsdatensatz zu ermitteln, welche die wenigsten eine erste Analysegröße umfasst, die die (bevorzugt physiko-chemische) Eigenschaft der ersten Füllstoffzusammensetzung und/oder der ersten Kompositmasse und/oder des ersten Gießwerkstücks charakterisiert.

Unter einem obig beschriebenen externen Server ist insbesondere ein in Bezug auf die Kompositmassenrezept-Ermittlungsvorrichtung gesehener externer Server, insbesondere ein Backend-Server, zu verstehen. Der externe Server ist beispielsweise ein Backend eines Produzenten für Füllstoffzusammensetzungen und/oder Kompositmassen und/oder Gießwerkstücken und/oder eines Händlers für Kompositmassen und/oder Gießwerkstücke, Rohstoffanbieter für Produkte in der Gießwerkstückindustrie oder eines Dienstanbieters, welcher dazu eingerichtet ist, Zusammensetzungsdatensätze in Bezug auf Füllstoffzusammensetzungen und/oder Kompositmassen und/oder Gießwerkstücke zu ermitteln und/oder zu erzeugen und/oder zu verwalten und/oder zu speichern. Die Funktionen des Backend bzw. des externen Servers können dabei auf (externen) Serverfarmen durchgeführt werden. Beim (externen) Server kann es sich um ein verteiltes System handeln. Der externe Server und/oder das Backend kann Cloud-basiert sein.

Die vorliegende Erfindung ist weiterhin gerichtet auf eine Anlage zur Herstellung einer Kompositmasse und/oder eines Gießwerkstücks, umfassend eine obig beschriebene Kompositmassenrezept-Ermittlungsvorrichtung entsprechend einer (obig beschriebenen) Ausführungsform.

Bevorzugt ist die Anlage mit einer entsprechenden Schnittstelle ausgerüstet, welche die Übermittlung des mindestens einen Zusammensetzungsdatensatzes und/oder die Auswahl einer gewünschten Analysegröße, die die (Soll-) Eigenschaft der Füllstoffzusammensetzung und/oder der Kompositmasse und/oder des Gießwerkstücks charakterisiert, ermöglicht.

Die vorliegende Erfindung ist weiterhin gerichtet auf ein Computerprogramm oder Computerprogrammprodukt, umfassend Programmmittel, insbesondere einen Programmcode, welcher zumindest einige der und bevorzugt alle Verfahrensschritte der erfindungsgemäßen Verfahren (Verfahren zur Erzeugung eines ersten Zusammensetzungsdatensatzes, welcher Daten für die Rezeptierung einer ersten Füllstoffzusammensetzung und/oder einer ersten Kompositmasse für die Herstellung eines ersten Gießwerkstücks umfasst; Verfahren zur Erzeugung eines trainierten Kompositmassenrezeptierungsmodells maschinellen Lernens; Verwendung des erzeugten trainierten Kompositmassenrezeptierungsmodells maschinellen Lernens zur Ermittlung wenigstens einer für wenigstens eine Eigenschaft einer ersten Füllstoffzusammensetzung und/oder einer ersten Kompositmasse und/oder einem ersten Gießwerkstück charakteristischen Analysegröße) und bevorzugt eine der beschriebenen bevorzugten Ausführungsformen repräsentiert oder kodiert und zum Ausführen durch eine Prozessoreinrichtung ausgebildet ist.

Die vorliegende Erfindung ist weiterhin gerichtet auf einen Datenspeicher, auf welchem zumindest eine Ausführungsform des erfindungsgemäßen Computerprogramms oder einer bevorzugten Ausführungsform des Computerprogramms gespeichert ist.

Weitere Vorteile und Ausführungsformen ergeben sich aus den beigefügten Zeichnungen:
Darin zeigt:
- Fig. 1: eine schematische Darstellung eines Ablaufs eines erfindungsgemäßen Verfahrens gemäß einer bevorzugten Ausführungsform und
- Fig. 2: eine schematische Darstellung eines mehrstufigen Ablaufs eines erfindungsgemäßen Verfahrens gemäß einer bevorzugten Ausführungsform.

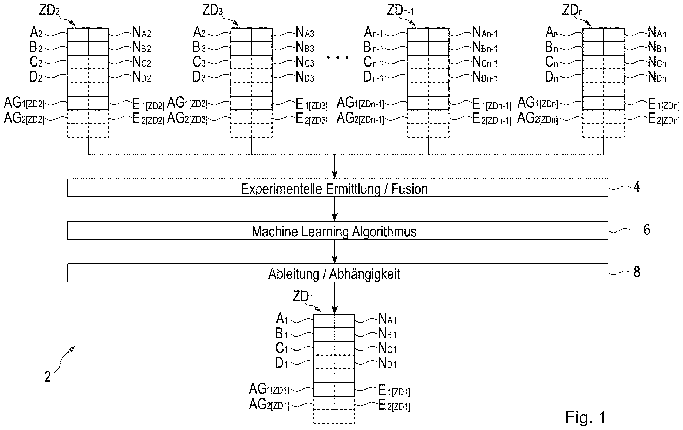

Fig. 1 zeigt eine schematische Darstellung eines Ablaufs eines erfindungsgemäßen Verfahrens (2) gemäß einer bevorzugten Ausführungsform. Die Bezugszeichen ZD₁ - ZDₙ kennzeichnen darin jeweils einen Zusammensetzungsdatensatz, ohne diese dahingehend zu spezifizieren, ob der jeweilige Zusammensetzungsdatensatz eine Füllstoffzusammensetzung, eine Kompositmasse oder ein Gießwerkstück charakterisiert. Jeder der Zusammensetzungsdatensätze ZD₁ - ZDₙ umfasst somit Deskriptoren für jeweils eine Füllstoffzusammensetzung, eine Kompositmasse oder ein Gießwerkstück. Diese Deskriptoren können durch Analyse erhalten werden oder aus der Rezeptur der jeweiligen Füllstoffzusammensetzung, Kompositmasse oder Gießwerkstücks bekannt sein. Die Anzahl, die Art und der Wert der Deskriptoren kann zwischen zwei Zusammensetzungsdatensätzen verschieden oder gleich sein.

Wie in Fig. 1 dargestellt ist, ist es Ziel des Verfahrens, die Deskriptoren für einen ersten Zusammensetzungsdatensatz ZD₁ zu bestimmen, welcher Daten für die Rezeptierung einer ersten Füllstoffzusammensetzung, einer ersten Kompositmasse oder eines ersten Gießwerkstücks umfasst.

Der erste Zusammensetzungsdatensatz ZD₁ umfasst eine Information zu einer ersten Komponente A₁ und wenigstens einer weiteren, von der ersten Komponente A₁ verschiedenen zweite Komponente B₁, sowie eine Anteilsgröße N_{A1} der ersten Komponente A₁ und eine Anteilsgröße N_{B1} der zweiten Komponente B₁. Die Anteilsgröße N_{A1}, N_{B1}, ... N_{An} steht dabei jeweils für den Anteil der Komponente A₁, B₁,... X₁ in der ersten Füllstoffzusammensetzung, der ersten Kompositmasse und/oder dem ersten Gießwerkstück.

Analog umfasst auch jeder der weiteren Zusammensetzungsdatensätze ZD₂ - ZDₙ eine Information zu einer ersten Komponente A₂ und wenigstens einer weiteren, von der ersten Komponente A₂ verschiedenen zweite Komponente B₂, sowie eine Anteilsgröße N_{A2} der ersten Komponente A₂ und eine Anteilsgröße N_{B2} der zweiten Komponente B₂. Die in einem Zusammensetzungsdatensatz ZD₂ - ZDₙ hinterlegten Komponenten A₂₋ₙ - X₂₋ₙ können dabei gleich oder verschieden sein. Denkbar wäre beispielsweise, dass eine erste Komponente in einem der Zusammensetzungsdatensätze ZD₂ - ZDₙ Quarz einer definierten Partikelgrößenverteilung als einen möglichen Füllstoff einer Füllstoffzusammensetzung, einer Kompositmasse und/oder einem Gießwerkstück definiert, Quarz dieser Partikelgrößenverteilung aber nicht in einer anderen der Füllstoffzusammensetzungen, einer Kompositmassen und/oder Gießwerkstücke enthalten ist. Dies könnte dadurch abgebildet werden, dass die Variable der Komponente Aₓ in einem anderen der Zusammensetzungsdatensätze ZD₂ - ZDₙ anders belegt ist als in dem Zusammensetzungsdatensatz ZD₁ oder dass die Variable der Komponente Aₓ in allen Zusammensetzungsdatensätzen ZD₁ - ZDₙ gleich belegt ist, jedoch die Anteilsgröße N_{Ax} für einen oder einige der Zusammensetzungsdatensätze ZD₁ - ZDₙ die Abwesenheit der Komponente Aₓ in der/dem durch diesen Zusammensetzungsdatensatz ZD₁ beschriebenen Füllstoffzusammensetzung, Kompositmasse und/oder Gießwerkstück durch einen Wert charakterisiert, der für einen Massenanteil von 0 steht.

Die Anzahl der Zusammensetzungsdatensätze ZD₂ - ZDₙ ist nach oben offen. Eine große Anzahl ist jedoch vorteilhaft, um die Robustheit des Verfahrens zu verbessern. Es hat sich aber gezeigt, dass bereits ein Paket von mehr als 50 Zusammensetzungsdatensätzen ZD₂ - ZDₙ (n = 50) ausreichend sein kann, um annehmbar gute Ergebnisse zu erreichen. Eine größere Anzahl von Analysedatensätzen wie beispielsweise ≥ 150 Analysedatensätze (n ≥ 150) ist jedoch wie oben dargelegt vorteilhaft im Hinblick auf die Qualität und Robustheit einer Berechnung beziehungsweise Vorhersage einer Eigenschaft einer Füllstoffzusammensetzung, Kompositmasse oder einem Gießwerkstück.

Der Zusammensetzungsdatensatz ZD₁ umfasst eine Information zu mindestens einer durch eine erste Analysegröße AG₁, AG₂, ... AGₙ charakterisierbaren Eigenschaft E₁, E₂, ... Eₙ der ersten Füllstoffzusammensetzung und/oder der ersten Kompositmasse und/oder des ersten Gießwerkstücks, wobei in Fig. 1 nicht spezifiziert ist, ob der Zusammensetzungsdatensatz ZD₁ eine Füllstoffzusammensetzung, eine Kompositmasse oder ein Gießwerkstück definiert.

Analog dazu umfasst auch mindestens ein zweiter und/oder weiterer Zusammensetzungsdatensatz ZD₂, ZD₃,... ZDₙ zu einer/einem zweiten und/oder weiteren Füllstoffzusammensetzung Kompositmasse oder Gießwerkstück jeweils eine die zweite und/oder weitere Analysegröße AG_{1-n[ZD2-ZDn]} jeweils charakteristisch ist für wenigstens eine Eigenschaft E_{1-n[FM2-FMn]}, E_{1-n[KM2-KMn],} E_{2-n[GWS2-GWSn]} der/des durch den jeweiligen Zusammensetzungsdatensatz ZD₂, ... ZDₙ charakterisierten Füllstoffzusammensetzung FM₂, ... FMₙ)und/ oder Kompositmasse KM₂, ... KMₙ und/oder Gießwerkstücks GWS₂, ... GWSₙ. Es ist dabei nicht notwendig, dass eine Analysegröße AG_{1-n[ZD2-ZDn]}, welche zu einer bestimmten Eigenschaft korreliert, tatsächlich in allen Zusammensetzungsdatensätzen ZD₂ - ZDₙ enthalten ist. denkbar wäre auch, dass für die weiteren Verfahrensschritte lediglich (oder mit höherer Gewichtung) diejenigen Zusammensetzungsdatensätze ZD₂ - ZDₙ berücksichtig werden, die eine Analysegröße AG_{1-n[ZD2-ZDn]} enthalten, die zu einer bestimmten (Soll-) Eigenschaft E_{1-n[ZD1-ZDn]} korreliert.

Jeder der Zusammensetzungsdatensätzen ZD₁ - ZDₙ umfasst somit bevorzugt mindestens eine Analysegröße AG_{1[ZD2-ZDn]}. Mehrere Analysegrößen AG_{1-n[ZD2-ZDn]} sind wie oben dargelegt vorteilhaft, wobei in Fig. 1 für jeden Analysedatensatz lediglich eine weitere Analysegröße AG_{2[ZD2-ZDn]} dargestellt ist und weitere Analysegrößen AG_{3-n[ZD2-ZDn]} lediglich durch gestrichelte Bereiche angedeutet sind.

Eine Analysegröße AG_{1-n[ZD2-ZDn]} steht jeweils für eine Eigenschaft E_{1-n[ZD1-n]} einer Füllstoffzusammensetzung, Kompositmasse oder eines Gießwerkstücks. Beispielsweise kann es sich dabei um einen Farbort, eine Härte, eine Viskosität, eine Dichte, eine Abriebbeständigkeit oder eine andere der oben genannten Eigenschaften handeln. Diese Eigenschaft kann bereits bei der Erstellung der Zusammensetzungsdatensätze ZD₁ - ZDₙ bekannt sein und in diesen enthalten sein, oder wird dem jeweiligen Zusammensetzungsdatensatz ZD₁ - ZDₙ nachträglich, beispielsweise nach einer entsprechenden Analyse der betreffenden Füllstoffzusammensetzung, Kompositmasse oder des Gießwerkstücks auf diese Eigenschaft hin, zugefügt. Es ist dabei jedoch nicht notwendig, dass jeder Zusammensetzungsdatensatz ZD₁ - ZDₙ zu jeder Eigenschaft E_{1-n[ZD1-n]} einen Wert enthält. Es ist auch denkbar, dass einzelne der Zusammensetzungsdatensätze ZD₁ - ZDₙ zu einer bestimmten Eigenschaft E_{1-n[ZD1-n]} keinen Wert enthalten.

Die oben genutzten Indizes "FM₁-FMₙ", "KM₁-KMₙ" und GWS₁-GWSₙ" stehen dabei für eine erste bis n-te Füllstoffzusammensetzung (FM1-FMn), erste bis n-te Kompositmasse (KM1-KMn) bzw. ein erstes bis n-tes Gießwerkstück (GWS₁, ... GWSₙ). Ist es unerheblich, ob es sich um eine Füllstoffzusammensetzung, eine Kompositmasse oder ein Gießwerkstück handelt oder wenn die obige Beschreibung allgemeingültig ist, wird der Index "ZD₁-ZDₙ" genutzt.

Der Index ZD₁-ZDₙ steht dann allgemein für eine erste bis n-te Füllstoffzusammensetzung, eine erste bis n-te Kompositmasse oder ein erstes bis n-tes Gießwerkstück.

Es wird vorgeschlagen, die Zusammensetzungsdatensätze ZD₂ - ZDₙ beziehungsweise die darin enthaltenen Deskriptoren (A₂₋ₙ, B₂₋ₙ, ...; N_{A2-n}, N_{B2-n[ZD1-ZDn]}, ...) einschließlich der Analysegröße AG_{1[ZD2-ZDn]} - AG_{n[ZD2-ZDn]} für einen Machine-Learning-Algorithmus 6 zu verwenden. Hierzu ist in dem mit dem Bezugszeichen 4 gekennzeichneten Verfahrensschritt eine Experimentelle Ermittlung/Fusion der Zusammensetzungsdatensätze ZD₂ - ZDₙ und der Analysegröße AG_{1[ZD2-ZDn]} - AG_{n[ZD2-ZDn]} vorgesehen.

Mit einer Anzahl an Trainingsdatensätzen wird nun der Machine-Learning-Algorithmus 6 trainiert und für den jeweiligen Zielwert, welcher zu einer Eigenschaft E_{1[ZD1]}, E_{2[ZD1]}, ...; E_{n[ZD1]} der ersten Füllstoffzusammensetzung FM₁, ersten Kompositmasse KM₁ oder des ersten Gießwerkstücks GWS₁ korreliert, eine Ableitungsfunktion in Abhängigkeit von den ermittelten Parametern erzeugt. In Fig. 1 kennzeichnet das Bezugszeichen 8 die Ableitung einer derartigen Abhängigkeit.

Der Trainingsdatensatz wird zum Training des Machine-Learning-Algorithmus 6 bzw. zum Trainieren eines neuronalen Netzwerkes bzw. zum maschinellen Lernen zur Vorhersage der physiko-chemischen Eigenschaft E_{1[ZD1]}, E_{2[ZD1]}, ...; E_{n[ZD1]} der ersten Füllstoffzusammensetzung FM₁, ersten Kompositmasse KM₁ oder des ersten Gießwerkstücks GWS₁ als Funktion der Zusammensetzungsdatensätze ZD₂, ... ZDₙ verwendet. Hieraus (durch das trainierte neuronale Netz bzw. durch den trainierten Machine-Learning-Algorithmus) ergibt sich insbesondere eine Vorhersagefunktion, welche bevorzugt zur Vorhersage wenigstens einer physiko-chemischen Eigenschaft E_{1[ZD1]}, E_{2[ZD1]}, ...; E_{n[ZD1]} der ersten Füllstoffzusammensetzung FM₁, ersten Kompositmasse KM₁ oder des ersten Gießwerkstücks GWS₁ verwendet werden kann. Aus dem zu der ersten Füllstoffzusammensetzung FM₁, ersten Kompositmasse KM₁ oder des ersten Gießwerkstücks GWS₁ korrelierenden Zusammensetzungsdatensatzes ZD₁ und der Vorhersagefunktion kann insbesondere ein Erwartungswert zu der wenigstens einen physiko-chemischen Eigenschaft E_{1[ZD1]}, E_{2[ZD1]}, ...; E_{n[ZD1]} dieser ersten Füllstoffzusammensetzung FM₁, ersten Kompositmasse KM₁ oder des ersten Gießwerkstücks GWS₁ ermittelt werden.

Ebenso ist es denkbar, einen Soll-Wert der physiko-chemischen Eigenschaft E_{1[ZD1]} vorzugeben und das System Deskriptoren für jeweils eine Füllstoffzusammensetzung, Kompositmasse oder ein Gießwerkstück ermitteln zu lassen welche gemäß dieser Vorhersage wahrscheinlich die vorgegebene Eigenschaft E_{1[ZD1]} aufweist. Bei dieser Variante des Verfahrens wird somit ein Soll-Wert der physiko-chemischen Eigenschaft E_{1[ZD1]} vorgegeben und die Komponenten (A₁, B₁, C₁,...) sowie die diesen Komponenten zugehörigen Anteilsgrößen (N_{A1}, N_{B1}, N_{C1}, ...) ermittelt. Eine Beschichtungszusammensetzung mit den Beschichtungszusammensetzungskomponenten (A_{Z}, B_{Z}, C_{Z,}...) in den Mengenanteilen gemäß der Anteilsgrößen (N_{Az}, N_{Bz}, N_{Cz}, ...) wird gemäß dieser Vorhersage die vorgegebene physiko-chemischen Eigenschaft E_{1[ZD1]} mit dem Soll-Wert aufweisen.

Basierend auf zumindest einigen der oben beschriebenen zweiten Zusammensetzungsdatensatzes ZD₂ und mindestens einem der weiteren Zusammensetzungsdatensätze ZD₃, ... ZDₙ erfolgt in dieser Verfahrensvariante eine Computer-implementierte Ermittlung von wenigstens einem Wert des ersten Zusammensetzungsdatensatzes ZD₁. Der mindestens eine dabei ermittelte Wert ist charakteristisch für
a) das Vorhandensein oder Nicht-Vorhandensein einer ersten Komponente (A₁) und/oder der wenigstens einen weiteren Komponente B₁ in der ersten Füllstoffzusammensetzung FM₁ und/oder einer ersten Kompositmasse KM₁ und/oder dem ersten Gießwerkstück GWS₁,
b) eine Anteilsgröße N_{A1}, N_{B1}, ... N_{X1} der ersten und/oder weiteren Komponente A₁, B₁, ... X₁ in der ersten Füllstoffzusammensetzung FM₁ und/oder einer ersten Kompositmasse KM₁ und/oder dem ersten Gießwerkstück GWS₁, oder
c) mindestens einen durch die erste Analysegröße AG_{1[ZD1]}, AG_{2[ZD1]}, ...; AG_{n[ZD1]} charakterisierbaren ersten Eigenschaft (E_{1[ZD1]}, E_{2[ZD1]}, ...; E_{n[ZD1]} der ersten Füllstoffzusammensetzung FM₁, ersten Kompositmasse KM₁ oder des ersten Gießwerkstücks GWS₁.

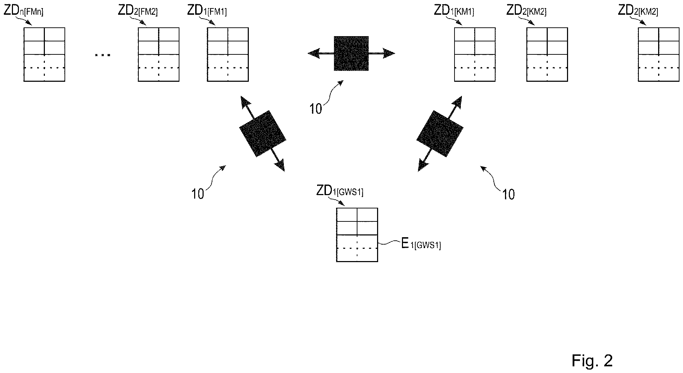

Fig. 2 zeigt eine schematische Darstellung eines mehrstufigen Ablaufs eines erfindungsgemäßen Verfahrens gemäß einer bevorzugten Ausführungsform. Dabei ist jeweils der Schritt der Computer-implementierte Ermittlung von wenigstens einem Wert eines Zusammensetzungsdatensatzes ZD_{1[FM1/KM1/GWS1]} schematisch dargestellt und mit Bezugszeichen 10 gekennzeichnet. Der Doppelpfeil soll dabei anzeigen, dass das Verfahren zur Ermittlung von Komponenten und/oder Eigenschaften beidseits des Doppelpfeils genutzt werden kann. So könnten beispielsweise Komponenten einer ersten Kompositmasse KM₁ vorgegeben werden und eine Eigenschaft E_{1[GWS1]} eines daraus herstellbaren Gießwerkstücks GWS₁ ermittelt werden.

Alternativ wäre wie oben dargelegt auch möchte, eine gewünschte (Soll-) Eigenschaft E₁, beispielsweise eines Gießwerkstück GWS₁ vorzugeben und diejenigen Komponenten einer ersten Kompositmasse KM₁ ermitteln zu lassen, die zu einem Gießwerkstück GWS₁ mit der gewünschten (Soll-) Eigenschaft E₁ umgesetzt werden können.

Ebenso wäre denkbar, dass eine gewünschte (Soll-) Eigenschaft E₁ beispielsweise eines Gießwerkstück GWS₁ vorgegeben wird und eine ersten Füllstoffzusammensetzung FM₁ ermittelt wird, die im Rahmen einer bestimmten Rezeptur und/oder eines bestimmten Verfahrens zu dem Gießwerkstück GWS₁ mit der gewünschten (Soll-) Eigenschaft E₁ umgesetzt werden kann.

Das Verfahren, kann auch dazu genutzt werden, um Verfahrensschritte zu bestimmen, die bei der Umsetzung einer Kompositmasse KM₁ zu einem Gießwerkstück GWS₁, einer Füllstoffzusammensetzung FM₁ zu einem Gießwerkstück GWS₁, oder einer Füllstoffzusammensetzung FM₁ zu einer Kompositmasse KM₁ notwendig sind, damit das Produkt die gewünschte (Soll-) Eigenschaft E₁ aufweist. Diese Ebenfalls durch ein Computer-implementiertes Verfahren ermittelbaren Verfahrensdaten sind in Fig. 2 schematisch durch das schwarte Quadrat im Bereich der Doppelpfeile symbolisiert.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in der Figur auch Merkmale beschrieben wurden, welche für sich genommen vorteilhaft sein können. Der Fachmann erkennt unmittelbar, dass ein bestimmtes in den Figuren beschriebenes Merkmal auch ohne die Übernahme weiterer Merkmale aus dieser Figur vorteilhaft sein kann. Ferner erkennt der Fachmann, dass sich auch Vorteile durch eine Kombination eines oder mehrerer in einer Figur gezeigter Merkmale mit einem oder mehreren im Text beschriebener Merkmale ergeben können.

## Patentansprüche

1. Verfahren zur Erzeugung eines ersten Zusammensetzungsdatensatzes (ZD₁), welcher Daten für die Rezeptierung einer ersten Füllstoffzusammensetzung (FM₁) und/oder einer ersten Kompositmasse (KM₁) für die Herstellung eines ersten Gießwerkstücks (GWS₁) umfasst, wobei der erste Zusammensetzungsdatensatz (ZD1) umfasst:
- eine Information zu einer ersten Komponente (A₁) und wenigstens einer weiteren, von der ersten Komponente (A₁) verschiedenen zweite Komponente (B₁),
- eine Anteilsgröße (N_{A1}) der ersten Komponente (A₁) und eine Anteilsgröße (N_{B1}) der zweiten Komponente (B₁) in der ersten Füllstoffzusammensetzung (FM₁) und/oder einer ersten Kompositmasse (KM₁) und/oder dem ersten Gießwerkstück (GWS₁), und
- eine Information zu mindestens einer durch eine erste Analysegröße (AG_{1[KM1]}, AG_{2[KM1]}, ...; AG_{1[GWS1]}, AG_{2[GWS1]}, ...) charakterisierbaren Eigenschaft (E_{1[KM1]}, E_{2[KM1]}, ...; E_{1[GWS1],} E_{2[GWS1]}, ...) der ersten Füllstoffzusammensetzung (FM₁) und/oder der ersten Kompositmasse (KM₁) und/oder des ersten Gießwerkstücks (GWS₁),
wobei das Verfahren die Schritte umfasst:
- Erfassung eines zweiten Zusammensetzungsdatensatzes (ZD₂) zu einer zweiten Füllstoffzusammensetzung (FM₂) und/oder einer zweiten Kompositmasse (KM₂) und/oder einem zweiten Gießwerkstück (GWS₂),
wobei der zweite Zusammensetzungsdatensatz (ZD₂) wenigstens eine zweite Analysegröße (AG_{1[KM2]}, AG_{1[GWS2]}) und eine Anteilsgröße (N_{A2}) einer zweiten Komponente (A₂) und bevorzugt einer Vielzahl von Anteilsgrößen (N_{B2}, N_{C2},...) von weiteren Komponenten (B₂, C₂,...) umfasst,
- Erfassung mindestens eines weiteren Zusammensetzungsdatensatzes (ZD₃, ... ZDₙ) zu mindestens einer weiteren Füllstoffzusammensetzung (FM₃, ... FMₙ) und/oder einer weiteren Kompositmasse (KM₃, ... KMₙ) und/oder einem weiteren Gießwerkstück (GWS₃, ... GWSₙ), wobei der mindestens eine weitere Zusammensetzungsdatensatz (ZD₃, ... ZDₙ) wenigstens eine weitere Analysegröße (AG_{1-n[FM3-FMn]}, AG_{1-n[KM3-KMn]}, AG_{1-n[GWS3-GWSn]}) und eine weitere Anteilsgröße (N_{A3}, ... N_{An}) einer weiteren Komponente (A₃, ... Aₙ) und bevorzugt eine Vielzahl von weiteren Anteilsgrößen (N_{B3}, ... N_{Bn}, N_{C3}, ... N_{Cn}, ...) von weiteren Komponenten (B₃₋ₙ, C₃₋ₙ, ...) umfasst,
wobei die zweite und/oder weitere Analysegröße (AG_{2-n[FM3-FMn]}, AG_{2-n[KM3-KMn]}, AG_{2-n[GWS3-GWSn]}) jeweils charakteristisch ist für wenigstens eine Eigenschaft (E_{2-n[KM3-KMn]}, E_{2-n[GWS3-GWSn]}) der/des durch den jeweiligen Zusammensetzungsdatensatz (ZD₂, ... ZDₙ) charakterisierten Füllstoffzusammensetzung (FM₂, ... FMₙ) und/ oder Kompositmasse (KM₂, ... KMₙ) und/oder Gießwerkstücks (GWS₂, ... GWSₙ),
wobei die zweite und/oder weitere Anteilsgröße (N_{A2}, ... N_{An}, N_{B2}, ... N_{Bn}, ...) jeweils charakteristisch ist für einen Mengenanteil der in der/dem durch den jeweiligen Zusammensetzungsdatensatz (ZD₂, ... ZDₙ) charakterisierten Füllstoffzusammensetzung (FM₂, ... FMₙ) und/oder Kompositmasse (KM₂, ... KMₙ) und/oder Gießwerkstück (GWS₂, ... GWSₙ) befindlichen ersten und/oder weiteren Komponente (A₂₋ₙ, B₂₋ₙ, ...); und
- Computer-implementierte Ermittlung von wenigstens einem Wert des ersten Zusammensetzungsdatensatzes (ZD₁) auf Grundlage des zweiten Zusammensetzungsdatensatzes (ZD₂) und mindestens eines weiteren Zusammensetzungsdatensatzes (ZD₃, ... ZDₙ), wobei der ermittelte Wert ausgewählt ist aus einer Information aus einer Gruppe von Informationen
a) über das Vorhandensein oder Nicht-Vorhandensein einer ersten Komponente (A₁) und/oder der wenigstens einen weiteren Komponente (B₁) in der ersten Füllstoffzusammensetzung (FM₁) und/oder einer ersten Kompositmasse (KM₁) und/oder dem ersten Gießwerkstück (GWS₁),
b) über eine Anteilsgröße (N_{A1}, N_{B1}, ...) der ersten und/oder weiteren Komponente (A₁, B₁, ...) in der ersten Füllstoffzusammensetzung (FM₁) und/oder einer ersten Kompositmasse (KM₁) und/oder dem ersten Gießwerkstück (GWS₁), und
c) zu der mindestens einen durch die erste Analysegröße (AG_{1[KM1]}, AG_{2[KM1]}, ...; **AG_{1[Gws1]}, AG_{2[GWS1],}** ...) charakterisierbaren ersten Eigenschaft (E_{1[KM1]}, E_{2[KM1]}, ...; **E**_{1[GWS1]}, **E_{2[GWS1]},** ...) der ersten Füllstoffzusammensetzung (FM₁) und/oder der ersten Kompositmasse (KM₁) und/oder des ersten Gießwerkstücks (GWS₁).

2. Verfahren nach Anspruch 1,
**dadurch gekennzeichnet, dass**
die mindestens eine erste Analysegröße (AG_{1[KM1]}, AG_{2[KM1]}, ...; AG_{1[GWS1]}, AG_{2[GWS1]}, ...), welche die erste Eigenschaft (E_{1[KM1]}, E_{2[KM1]}, ...; E_{1[Gws1]}, E_{2[GWS1]}, ...) charakterisiert für die erste Füllstoffzusammensetzung (FM₁) und/oder die erste Kompositmasse (KM₁) und/oder das erste Gießwerkstück (GWS₁) vorgegeben wird und während der computer-implementierten Ermittlung des wenigstens einen Wertes des ersten Zusammensetzungsdatensatzes (ZD₁)
a) das Vorhandensein oder Nicht-Vorhandensein einer ersten Komponente (A₁) und/oder der wenigstens einen weiteren Komponente (B₁) in der ersten Füllstoffzusammensetzung (FM₁) und/oder in der ersten Kompositmasse (KM₁) und/oder dem ersten Gießwerkstück (GWS₁), und
b) eine Anteilsgröße (N_{A1}, N_{B1}, ...) der ersten und/oder weiteren Komponente (A₁, B₁, ...) in der ersten Füllstoffzusammensetzung (FM₁) und/oder der ersten Kompositmasse (KM₁) und/oder dem ersten Gießwerkstück (GWS₁)
ermittelt wird.

3. Verfahren nach einem der vorhergehenden Ansprüche,
**dadurch gekennzeiehnet, dass**
mindestens eine der ersten Komponente (A₁) oder der wenigstens einen weiteren Komponente (B₁, C₁, ...) und eine andere der ersten Komponente (A₁) oder der wenigstens einen weiteren Komponente (B₁, C₁, ...) unabhängig voneinander ausgewählt ist aus einer Gruppe, die ein Rheologieadditiv, ein Pigment, einen Farbstoff, eine Farbpaste, ein Effektpigment, einen Emulgator, ein Biozid, einen Härter, einen Beschleuniger, einen Inhibitor, einen Füllstoff, ein Lösungsmittel, ein Polymer, ein Monomer, einen Polymerisationsstarter, ein Organosilan, einen Quervernetzer, einen Entschäumer, ein Benetzungsadditiv, ein Quarz- und/oder Quarzfolgeprodukt, eine Ca- und/oder Mg-haltige Komponente, eine Al-haltige Komponente, ein Konservierungsmittel, insbesondere ein Film- und/oder Topfkonservierungsmittel, einen anorganischen und/oder organischen Füllstoff umfasst, der sowohl mineralisch, amorph, oder biogen vorlegen kann wobei bevorzugt mindestens eine weitere der ersten Komponente (A₁) oder der wenigstens eine weiteren Komponente (B₁, C₁, ...) ausgewählt ist aus einer Gruppe, die ein Rheologieadditiv, ein Pigment, einen Farbstoff, eine Farbpaste, ein Effektpigment, einen Emulgator, ein Biozid, einen Härter, einen Beschleuniger, einen Inhibitor, einen Füllstoff, ein Lösungsmittel, ein Polymer, ein Monomer, einen Polymerisationsstarter, ein Organosilan, einen Quervernetzer, einen Entschäumer, ein Benetzungsadditiv, ein Quarz- und/oder Quarzfolgeprodukt, eine Ca- und/oder Mg-haltige Komponente, eine Al-haltige Komponente, ein Konservierungsmittel, insbesondere ein Film- und/oder Topfkonservierungsmittel, einen anorganischen und/oder organischen Füllstoff umfasst, der sowohl mineralisch, amorph, oder biogen vorlegen kann.

4. Verfahren nach einem der vorhergehenden Ansprüche,
**dadurch gekennzeiehnet, dass**
das erste Gießwerkstück (GWS₁) ausgewählt ist aus einer Gruppe, die eine Küchenspüle, eine Küchenarbeitsplatte, ein Waschbecken, eine Duschtasse, eine Duschkabine, eine Toilette, eine Badewanne, eine (Wand-) Fliese, eine (Boden-) Platte, einen Bodenbelag, ein Möbelteil und eine Trennwand umfasst.

5. Verfahren nach einem der vorhergehenden Ansprüche,
**dadurch gekennzeiehnet, dass**
das Verfahren weiterhin die Erzeugung eines ersten Verfahrensdatensatzes (VD₁) umfasst, der Daten betreffend ein Verfahren zur Umsetzung einer ersten Füllstoffzusammensetzung (FM₁) und/oder einer ersten Kompositmasse (KM₁) zu einem ersten Gießwerkstück (GWS₁) umfasst, wobei das Verfahren zur Erzeugung des ersten Verfahrensdatensatzes (VD₁) die Schritte umfasst:
- Erfassung eines zweiten Verfahrensdatensatzes (VD₂), der Daten betreffend ein Verfahren zur Umsetzung einer zweiten Füllstoffzusammensetzung (FM₂) zu einer Kompositmasse (KM₂) und zu einem zweiten Gießwerkstück (GWS₂) umfasst, wobei der zweite Verfahrensdatensatz (VD₂) mindestens einen Wert enthält, welcher für einen Prozessparameter charakteristisch ist, der einen Effekt auf die durch die erste Analysegröße (AG₁) charakterisierte erste Eigenschaft (E₁) einer Füllstoffzusammensetzung (FM_{X}) und/oder einer Kompositmasse (KMₓ) und/oder einem Gießwerkstück (GWSₓ) hat,
- Erfassung mindestens eines weiteren Verfahrensdatensatzes (VD₃, ... VDₙ), der jeweils Daten betreffend ein Verfahren zur Umsetzung einer weiteren Füllstoffzusammensetzung (FM₃, ... FMₙ) und/oder einer weiteren Kompositmasse (KM₃, ... KMₙ) zu einem weiteren Gießwerkstück (GWS₃, ... GWSₙ) umfasst, wobei der weitere Verfahrensdatensatz (VD₃, ... VDₙ) mindestens einen Wert enthält, welcher für einen Prozessparameter charakteristisch ist, der einen Effekt auf die durch die erste Analysegröße (AG₁) charakterisierte erste Eigenschaft (E₁) einer Füllstoffzusammensetzung (FMₓ) und/oder einer Kompositmasse (KMₓ) und/oder einem Gießwerkstück (GWSₓ) hat,
- Computer-implementierte Ermittlung von wenigstens einem Verfahrensdatenwert (VDW₁) des ersten Verfahrensdatensatzes(VD₁), auf Grundlage des zweiten Verfahrensdatensatzes (VD₂) und mindestens eines weiteren Verfahrensdatensatzes(VD₃, ... VDₙ), wobei dieser Verfahrensdatenwert (VDW₁) für einen Prozessparameter charakteristisch ist, dessen Einstellung von einem anderen Wert hin zu dem durch den Verfahrensdatenwert (VDW₁) charakterisierten Wert zumindest eine Annäherung der ersten Analysegröße (AG₁) hin zu einer Soll-Analysegröße ermöglicht, welche eine Soll-Eigenschaft des ersten Gießwerkstücks (GWS₁) charakterisiert.

6. Verfahren nach Anspruch 5,
**dadurch gekennzeiehnet, dass**
der während der computer-implementierten Ermittlung des wenigstens einen Verfahrensdatenwerts (VDW₁) des ersten Verfahrensdatensatzes (VD₁) ermittelte wenigstens eine Verfahrensdatenwerts (VDW₁) charakteristisch ist für einen Prozessparameter, der ausgewählt ist aus einer Gruppe, die eine Zeit, eine Temperatur, einen Druck, ein Werkzeug, eine Vorbehandlung eines Werkzeugs, eine Art einer Applikation eines Reagenzes und eine Reihenfolge der Zugabe von Reagenzien umfasst.

7. Verfahren nach Anspruch 5 oder 6,
**dadurch gekennzeiehnet, dass**
der zweite Verfahrensdatensatz (VD₂) und/oder mindestens einer der weiteren Verfahrensdatensätze (VD₃, ... VDₙ) jeweils mindestens 3 Verfahrensdatenwerte (VDWₓ), bevorzugt mindestens 5, weiter bevorzugt mindestens 7, meist bevorzugt mindestens 10 Verfahrensdatenwerte (VDWₓ) umfasst, die für einen Prozessparameter zur Umsetzung einer zweiten oder weiteren Füllstoffzusammensetzung (FM₂, FM₃, ...FMₙ) und/oder einer weiteren Kompositmasse (KM₂, KM₃, ... KMₙ) zu einem zweiten oder weiteren Gießwerkstück (GWS₂, GWS₃, ... GWSₙ) charakteristisch sind und welche bevorzugt während einer Umsetzung einer zweiten oder weiteren Füllstoffzusammensetzung (FM₂, FM₃, ...FMₙ) und/oder einer zweiten oder weiteren Kompositmasse (KM₂, KM₃, ... KMₙ) zu einem zweiten oder weiteren Gießwerkstück (GWS₂, GWS₃, ... GWSₙ) bestimmt wurden.

8. Ein Computer-implementiertes Verfahren zur Erzeugung eines trainierten Kompositmassenrezeptierungsmodells maschinellen Lernens zur Ermittlung wenigstens einer ersten Eigenschaft (E_{1[KM1]}, E_{2[KM1]}, ...; E_{1[GWS1],} E_{2[GWS1]}, ...) einer ersten Füllstoffzusammensetzung (FM₁) und/oder einer ersten Kompositmasse (KM₁) und/oder eines ersten daraus herstellbaren Gießwerkstück (GWS₁) auf Basis einer Verwendung von Zusammensetzungsdatensätzen (ZD₂ - ZDₙ), welche jeweils für eine Füllstoffzusammensetzung (FM₂, ...FMₙ) Kompositmasse (KM₂, ... KMₙ) und/oder ein Gießwerkstück (GWS₂, ... GWSₙ) charakteristisch sind, wobei der Zusammensetzungsdatensatz (ZD₂ - ZDₙ) zu der jeweiligen Füllstoffzusammensetzung (FM₂, ...FMₙ) und/oder der jeweiligen Kompositmasse (KM₂, ... KMₙ) und/oder dem jeweiligen Gießwerkstück (GWS₂, ... GWSₙ) jeweils wenigstens eine Analysegröße (AG_{2-n[KM2-KMn]}, AG_{2-n[GWS2-GWSn]}) und eine Anteilsgröße (N_{A2}, ... N_{An}, N_{B2}, ... N_{Bn}, N_{C2}, ... N_{Cn}, ...) einer Komponente (A₂, ... Aₙ, B₂₋ₙ, C₂₋ₙ, ...) umfasst, wobei die Analysegröße jeweils charakteristisch ist für wenigstens eine Eigenschaft (E_{2-n[KM3-KMn]}, E_{2-n[GWS3-GWSn]}) der/des durch den jeweiligen Zusammensetzungsdatensatz (ZD₂, ... ZDₙ) charakterisierten Füllstoffzusammensetzung (FM₂, ...FMₙ) und/oder der Kompositmasse (KM₂, ... KMₙ) und/oder Gießwerkstücks (GWS₂, ... GWSₙ), umfassend:
- Bereitstellen eines trainierbaren Kompositmassenrezeptierungsmodells maschinellen Lernens, welches einen Satz trainierbarer Parameter umfasst und welches auf Grundlage von zu mindestens einem zu einer Füllstoffzusammensetzung (FM₂, ...FMₙ) und/oder einer Kompositmasse (KM₂, ... KMₙ) und/oder einem Gießwerkstück (GWS₂, ... GWSₙ) erfassten Zusammensetzungsdatensatz (ZD₃, ... ZDₙ) oder hiervon abgeleiteter Daten als Eingangsgrößen wenigstens eine für eine Eigenschaft (E_{1[FM1]}, E_{2[FM1]}, ...;) der ersten Füllstoffzusammensetzung (FM₁) und/oder für eine Eigenschaft (E_{1[KM1]}, E_{2[KM1]}, ...;) der ersten Kompositmasse (KM₁) und/oder für eine Eigenschaft (E_{1[GWS1]}, E_{2[GWS1]}, ...) des ersten Gießwerkstück (GWS₁) charakteristische Analysegröße (AG_{1[FM1]}, AG_{2[FM1]}, ^{...;}AG_{1[KM1]}, AG_{2[KM1]}, ...; AG_{1GWS1]}, AG_{2[GWS1]}, ...) als Ausgangsgröße erzeugt;
- Erzeugen eines Trainingsdatensatzes umfassend eine Vielzahl erfasster und/oder ermittelter Zusammensetzungsdatensätze (ZD₂, ... ZDₙ) zu Trainings- Füllstoffzusammensetzungen (FM₁, ...FMₙ) und/oder von Trainings-Kompositmassen (KM₂, ... KMₙ) und/oder Trainings-Gießwerkstücken (GWS₂, ... GWSₙ), welche zum Erzeugen der Trainingsdatensätzen verwendet werden, sowie Messdaten oder eine hiervon abgeleitete Analysegrößen (AG_{2-n[KM2-KMn]}, AG_{2-n[GWS2-GWSn]}), welche charakteristisch sind bzw. ist für wenigstens eine Eigenschaft (E_{1-n[FM2-n]}, E_{2-n[FM2-n]}, ...; E_{1-n[KM2-n]}, E_{2-n[KM2-n]}, ...; E_{1-n[GWS2-n]}, E_{2-n[GWS2-n]}, ...) der jeweiligen Trainings-Füllstoffzusammensetzung (FM₂, ... FMₙ) und/oder der jeweiligen Trainings-Kompositmasse (KM₂, ... KMₙ) und/oder des Trainings-Gießwerkstücks (GWS₂, ... GWSₙ);
- Trainieren des Kompositmassenrezeptierungsmodells auf Grundlage des Trainingsdatensatzes.

9. Verwendung des nach dem vorhergehenden Anspruch erzeugten Kompositmassenrezeptierungsmodells zur Ermittlung wenigstens einer für wenigstens eine Eigenschaft (E_{1[FM1]}, E_{2[FM1]}, ...; E_{1[KM1]}, E_{2[KM1]}, ...; E_{1[GWS1],} E_{2[GWS1]}, ...) einer ersten Füllstoffzusammensetzung (FM₁) und/oder einer ersten Kompositmasse (KM₁) und/oder einem ersten Gießwerkstück (GWS₁) charakteristischen Analysegröße (AG_{1[FM1]}, AG_{2[FM1]}, ...;AG_{1[KM1]}, AG_{2[KM1]}, ...; AG_{1[GWS1]}, AG_{2[GWS1]}, ...).

10. Kompositmassenrezept-Ermittlungsvorrichtung zur Ermittlung wenigstens einer Eigenschaft (E_{1/FM1}), E_{2[FM1]}, ...; E_{1[KM1]}, E_{2[KM1]}, ...; E_{1[GWS1],} E_{2[GWS1]}, ...) einer ersten Füllstoffzusammensetzung (FM₁) und/oder einer ersten Kompositmasse (KM₁) und/oder einem ersten Gießwerkstück (GWS₁),
**dadurch gekennzeiehnet, dass**
die Kompositmassenrezept-Ermittlungsvorrichtung dazu geeignet und bestimmt ist, einen zweiten Zusammensetzungsdatensatz (ZD₂) zu einer zweiten Füllstoffzusammensetzung (FM₂) und/oder einer zweiten Kompositmasse (KM₂) und/oder einem zweiten Gießwerkstück (GWS₂) und mindestens einen weiteren Zusammensetzungsdatensatz (ZD₃, ... ZDₙ) zu mindestens einer weiteren Füllstoffzusammensetzung (FM₃, ... FMₙ) und/oder einer weiteren Kompositmasse (KM₃, ... KMₙ) und/oder einem weiteren Gießwerkstück (GWS₃, ... GWSₙ) zu erfassen, wobei der jeweilige Zusammensetzungsdatensatz (ZD₂, ZD₃, ... ZDₙ) wenigstens eine zweite oder weitere Analysegröße (AG_{2-n[FM3}-_{FMn]}, AG_{2-n[KM3-KMn]}, AG_{2-n[GWS3-GWSn]}) und wenigstens eine zweite oder weitere Anteilsgröße (N_{A2}, N_{A3}, ... N_{An}) einer zweiten oder weiteren Komponente (A₂, A₃, ... Aₙ) und bevorzugt einer Vielzahl von weiteren Anteilsgrößen (N_{B2}, ... N_{Bn}, N_{C2}, ... N_{Cn}, ...) von zweiten oder weiteren Komponenten (B₂₋ₙ, C₂₋ₙ, ...) umfasst, wobei die Analysegröße (AG_{2-n[FM3}-_{FMn]}, AG_{2-n[KM3-KMn]}, AG_{2-n[GWS3-GWSn]}) jeweils charakteristisch ist für wenigstens eine Eigenschaft (E_{1-n[FM2-n]}, E_{2-n[FM2-n]}, ...; E_{1-n[KM2-n]}, E_{2-n[KM2-n]}, ...; E_{1-n[GWS2-n]}, E_{2-n[GWS2-n]}, ...) einer zweiten oder weiteren Füllstoffzusammensetzung (FM₂₋ₙ) und/oder einer weiteren Kompositmasse (KM₂₋ₙ) und/oder einem zweiten oder weiteren Gießwerkstück (GWS₂₋ₙ), und wobei die Anteilsgröße (N_{B2}, ... N_{Bn}, N_{C2}, ... N_{Cn}, ...) jeweils charakteristisch ist für einen Mengenanteil der in der jeweiligen Füllstoffzusammensetzung (FM₂₋ₙ) und/oder der jeweiligen Kompositmasse (KM₂₋ₙ) und/oder dem jeweiligen Gießwerkstück (GWS₂₋ₙ) befindlichen zweiten und/oder weiteren zweiten oder weiteren Komponente (A₂, A₃, ... Aₙ), wobei die Kompositmassenrezept-Ermittlungsvorrichtung dazu geeignet und bestimmt ist, mittels einer Prozessoreinrichtung wenigstens einen Wert eines Zusammensetzungsdatensatzes (ZD1), welcher Daten für die Rezeptierung einer ersten Füllstoffzusammensetzung (FM₁) und/oder einer ersten Kompositmasse (KM₁) für die Herstellung eines ersten Gießwerkstück (GWS₁) umfasst, auf Grundlage des zweiten Zusammensetzungsdatensatz (ZD₂) zu der zweiten Füllstoffzusammensetzung (FM₂) und/oder der zweiten Kompositmasse (KM₂) und/oder dem zweiten Gießwerkstück (GWS₂) und dem mindestens einen weiteren Zusammensetzungsdatensatz (ZD₃, ... ZDₙ) zu der mindestens einen weiteren Füllstoffzusammensetzung (FM₃, ... FMₙ) und/oder einer weiteren Kompositmasse (KM₃, ... KMₙ) und/oder dem weiteren Gießwerkstück (GWS₃, ... GWSₙ) zu ermitteln.
